# EUROPEAN PATENT APPLICATION

(11) **EP 2 048 230 A1**
(43) Date of publication of application: **15.04.2009**
(21) Application number: 07790727.7
(22) Date of filing: 13.07.2007
(51) Int. Cl.: C12N 15/00, A61K 39/395, A61P 7/00, A61P 35/00, C07K 16/18, C12N 1/15, C12N 1/19, C12N 1/21, C12N 5/10, C12N 15/09, C12P 21/08

(54) **CELL DEATH INDUCER**

(30) Priority: 13.07.2006 JP 2006193053
(71) Applicant: CHUGAI SEIYAKU KABUSHIKI KAISHA, Tokyo, 115-8543 (JP)
(72) Inventor: KIMURA, Naoki, Gotenba-shi, Shizuoka 412-8513 (JP); KAWAI, Shigeto, Gotenba-shi, Shizuoka 412-8513 (JP)
(74) Representative: Vossius & Partner
(86) International application number: PCT/JP2007/063946
(87) International publication number: WO 2008/007755

(57) **Abstract**

An objective of the present invention is to provide an antibody having a high cell death-inducing activity. To solve the above-described problems, the present inventors immunized mice with cells expressing human HLA class IA and human β2 microglobulin (β2M) to obtain monoclonal antibodies. Screening of the obtained antibodies was performed to obtain ten clones of antibodies having a cell death-inducing activity. Analyses of these clones revealed that three of the clones (antibodies C3B3, C11B9, and C17D11), which have the α2 domain of the HLA class I antigen as an epitope, showed a stronger cytotoxic activity when crosslinked with an anti-mouse IgG antibody. Furthermore, when a C3B3 diabody was generated, this diabody was revealed to show a stronger anti-tumor effect compared with conventional diabodies of the 2D7 antibody, which is an HLA class IA antibody.

## Description

### Technical Field

The present invention relates to HLA-recognizing antibodies and cell death-inducing agents which comprise those antibodies as an active ingredient.

### Background Art

HLA is an important molecule in immune response which involves recognizing exogenous antigens, bacteria, virus-infected cells, and such as foreign substances and eliminating them. The main role of the HLA molecule is to present to CD8⁺T cells antigenic peptides produced inside cells, which are made up of about eight to ten amino acids, and thus, it plays a very important role in the immune response and immune tolerance induced by the peptide presentation. HLA molecules are categorized into class I and class II. Class I molecules form a heterodimer of a 12-KD β2 microglobulin (β2M) and a 45-KD α chain comprising three domains, α1-3. Class II molecules form a heterodimer of a 30-34 KD α-chain comprising two domains, α1 and α2, and a 26-29 KD β chain comprising two domains, β1 and β2. It is also known that HLA class I (HLA-I) can be further classified into HLA-A, B, C, and such (hereinafter, HLA-A is also called as "HLA class I A (HLA-IA)").

To date, cell growth-suppressing and cell death-inducing effects have been reported for lymphocytes that are ligated with an anti-HLA class IA antibody, suggesting that HLA molecules may be signal transduction molecules. For example, it has been reported that cell growth of activated lymphocytes is suppressed by B9.12.1 antibody against the α1 domain of human HLA class IA, W6/32 antibody against the α2 domain, and TP25.99 and A1.4 antibodies against the α3 domain (Non-Patent Documents 1 and 2). Furthermore, two antibodies against the α1 domain, MoAb90 and YTH862, have been reported to induce apoptosis in activated lymphocytes (Non-Patent Documents 2, 3, and 4). Apoptosis induced by these two antibodies has been shown to be a caspase-mediated reaction (Non-Patent Document 4), and therefore, HLA class IA expressed in lymphocytes is also speculated to be involved in apoptosis signal transduction.

Furthermore, 5H7 antibody against the α3 domain of human HLA class IA (Non-Patent Document 5), and RE2 antibody against the α2 domain of mouse MHC class I (Non-Patent Document 6) have been also reported to induce cell death in activated lymphocytes and the like.

Monoclonal antibody 2D7 (Non-Patent Document 9) obtained by immunizing human myeloma cells is also reported to be a HLA class IA-recognizing antibody, and can quickly induce severe cell death in human myeloma cells if made into a low-molecular antibody (diabody). The 2D7 diabody is under development as a therapeutic agent for myeloma, because it shows strong cell death-inducing activity in various human myeloma cell lines and activated lymphocytes, and demonstrates significant survival benefit in multiple myeloma model mice generated by transplanting a human myeloma cell into mice (Patent Documents 1, 2, 3, and 4, Non-Patent Documents 7 and 8). Further advances in treatments utilizing cell death involving HLA class I are expected to lead to development of highly effective pharmaceuticals against myeloma and the like.

Prior art literature relating to the present invention of this application is shown below.
[Patent Document 1] WO2004/033499
[Patent Document 2] WO2005/056603
[Patent Document 3] WO2005/100560
[Patent Document 4] PCT/JP2006/309890
[Non-Patent Document 1] Fayen et al., Int. Immunol. 10: 1347-1358(1998)
[Non-Patent Document 2] Genestier et al., Blood 90: 3629-3639 (1997)
[Non-Patent Document 3] Genestier et al., Blood 90: 726-735 (1997)
[Non-Patent Document 4] Genestier et al., J. Biol. Chem. 273: 5060-5066 (1998)
[Non-Patent Document 5] Woodle et al., J. Immunol. 158: 2156-2164 (1997)
[Non-Patent Document 6] Matsuoka et al., J. Exp. Med. 181: 2007-2015 (1995)
[Non-Patent Document 7] Goto, et al. Blood 84: 1922-30 (1994)
[Non-Patent Document 8] Kimura, et al. Biochem Biophys Res Commun., 325:1201-1209 (2004)
[Non-Patent Document 9] Oka, T., "Sankyo Seimei-kagaku-zaidan Kenkyu Hokoku-shu (Research Reports of the Sankyo Foundation of Life Science)" 12:46-56 (1998)

### Disclosure of the Invention

### [Problems to be Solved by the Invention]

The present invention was achieved in view of the above circumstances. An objective of the present invention is to provide antibodies comprising heavy chain variable regions that comprise CDR 1, 2, and 3 consisting of the amino acid sequences of SEQ ID NOs: 7, 8, and 9. Furthermore, an objective of the present invention is to provide antibodies comprising light chain variable regions that comprise CDR 1, 2, and 3 consisting of the amino acid sequences of SEQ ID NOs: 10, 11, and 12. More specifically, an objective of the present invention is to provide antibodies that recognize HLA class IA and have higher cell death-inducing activity than ever before.

### [Means for Solving the Problems]

The present inventors conducted dedicated research to solve the above-mentioned objectives. First, mice were immunized with cells co-expressing human HLA class IA and human β2M, and monoclonal antibodies were obtained. Then, the obtained antibodies were screened to obtain ten clones of new monoclonal antibodies having cell death-inducing activity. When these clones were analyzed, three clones (C3B3, C11B9, and C17D11 antibodies) whose epitope is an HLA class I antigen α2 domain, were found to show strong cellular cytotoxicity by cross-linking with an anti-mouse IgG antibody. Furthermore, by modifying the obtained C3B3 antibody to a low-molecular-weight antibody (C3B3 diabody), the present inventors succeeded in constructing a cell death-inducing agonistic antibody which by itself has an antitumor activity surpassing that of the conventional anti-HLA class IA low-molecular-weight antibody (2D7 diabody).

More specifically, the present invention provides the following [1] to [25]:
[1] an antibody comprising heavy chain variable regions that comprise CDR1, 2, and 3 consisting of the amino acid sequences of SEQ ID NOs 7, 8, and 9;
[2] an antibody comprising light chain variable regions that comprise CDR1, 2, and 3 consisting of the amino acid sequences of SEQ ID NOs 10, 11, and 12;
[3] an antibody comprising heavy chain variable regions that comprise CDR1, 2, and 3 consisting of the amino acid sequences of SEQ ID NOs 7, 8, and 9, and light chain variable regions that comprise CDR1, 2, and 3 consisting of the amino acid sequences of SEQ ID NOs 10, 11, and 12;
[4] an antibody comprising the heavy chain variable region of any one of:
   (a) a heavy chain variable region comprising the amino acid sequence of SEQ ID NO: 2;
   (b) a heavy chain variable region that comprises an amino acid sequence with one or more amino acid substitutions, deletions, insertions, and/or additions in the amino acid sequence of SEQ ID NO: 2, and which is functionally equivalent to the heavy chain variable region of (a);
   (c) a heavy chain variable region comprising an amino acid sequence encoded by a DNA comprising the nucleotide sequence of SEQ ID NO: 1; and
   (d) a heavy chain variable region comprising an amino acid sequence encoded by a DNA that hybridizes under stringent conditions with a DNA comprising the nucleotide sequence of SEQ ID NO: 1;
[5] an antibody comprising the light chain variable region of any one of:
   (e) a light chain variable region comprising the amino acid sequence of SEQ ID NO: 4;
   (f) a light chain variable region that comprises an amino acid sequence with one or more amino acid substitutions, deletions, insertions, and/or additions in the amino acid sequence of SEQ ID NO: 4, and which is functionally equivalent to the light chain variable region of (e);
   (g) a light chain variable region comprising an amino acid sequence encoded by a DNA comprising the nucleotide sequence of SEQ ID NO: 3; and
   (h) a light chain variable region comprising an amino acid sequence encoded by a DNA that hybridizes under stringent conditions with a DNA comprising the nucleotide sequence of SEQ ID NO: 3;
[6] an antibody comprising a heavy chain variable region of any one of:
   (a) a heavy chain variable region comprising the amino acid sequence of SEQ ID NO: 2;
   (b) a heavy chain variable region that comprises an amino acid sequence with one or more amino acid substitutions, deletions, insertions, and/or additions in the amino acid sequence of SEQ ID NO: 2, and which is functionally equivalent to the heavy chain variable region of (a);
   (c) a heavy chain variable region comprising an amino acid sequence encoded by a DNA comprising the nucleotide sequence of SEQ ID NO: 1; and
   (d) a heavy chain variable region comprising an amino acid sequence encoded by a DNA that hybridizes under stringent conditions with a DNA comprising the nucleotide sequence of SEQ ID NO: 1;
      and a light chain variable region of any one of:
   (e) a light chain variable region comprising the amino acid sequence of SEQ ID NO: 4;
   (f) a light chain variable region that comprises an amino acid sequence with one or more amino acid substitutions, deletions, insertions, and/or additions in the amino acid sequence of SEQ ID NO: 4, and which is functionally equivalent to the light chain variable region of (e);
   (g) a light chain variable region comprising an amino acid sequence encoded by a DNA comprising the nucleotide sequence of SEQ ID NO: 3; and
   (h) a light chain variable region comprising an amino acid sequence encoded by a DNA that hybridizes under stringent conditions with a DNA comprising the nucleotide sequence of SEQ ID NO: 3;
[7] an antibody comprising the amino acid sequence of any one of:
   (a) the amino acid sequence of SEQ ID NO: 6;
   (b) an amino acid sequence with one or more amino acid substitutions, deletions, insertions, and/or additions in the amino acid sequence of SEQ ID NO: 6;
   (c) an amino acid sequence encoded by a DNA comprising the nucleotide sequence of SEQ ID NO: 5; and
   (d) an amino acid sequence encoded by a DNA that hybridizes under stringent conditions with a DNA comprising the nucleotide sequence of SEQ ID NO: 5;
[8] an antibody that binds to a same epitope as the epitope of the human leukocyte antigen (HLA) protein to which the antibody of any one of [1] to [7] binds;
[9] the antibody of any one of [1] to [8], which is a monoclonal antibody.
[10] the antibody of any one of [1] to [9] that recognizes a human leukocyte antigen (HLA);
[11] the antibody of [10], wherein the HLA is an HLA class I;
[12] the antibody of [11], wherein the HLA class I is an HLA-A;
[13] the antibody of any one of [1] to [12], which is a low-molecular weight antibody;
[14] the antibody of [13], wherein the low-molecular-weight antibody is a diabody;
[15] a polynucleotide of (a) or (b):
   (a) a polynucleotide comprising the nucleotide sequence of SEQ ID NO: 1, 3, or 5; or
   (b) a polynucleotide that hybridizes under stringent conditions with the polynucleotide of (a), and encodes an antibody having an activity equivalent to the antibody of any one of [1] to [14];
[16] a vector comprising the polynucleotide of [15];
[17] a host cell that comprises the polynucleotide of [15] or the vector of [16];
[18] a method for producing the antibody of any one of [1] to [14], wherein the method comprises the steps of:
   (a) producing the polynucleotide of [15];
   (b) constructing a vector comprising the polynucleotide of (a);
   (c) introducing the vector of (b) into host cells; and
   (d) culturing the host cells of (c);
[19] a cell death-inducing agent comprising the antibody of any one of [1] to [14] as an active ingredient;
[20] the cell death-inducing agent of [19] which induces cell death of a B cell or T cell;
[21] the cell death-inducing agent of [20], wherein the B cell or T cell is an activated B cell or activated T cell;
[22] a cell growth-suppressing agent comprising the antibody of any one of [1] to [14] as an active ingredient;
[23] an antitumor agent comprising the antibody of any one of [1] to [14] as an active ingredient;
[24] the antitumor agent of [23], wherein the tumor is hematopoietic tumor; and
[25] a therapeutic agent for autoimmune diseases which comprises the antibody of any one of [1] to [14] as an active ingredient.

### Brief Description of the Drawings

Fig. 1 shows the results of confirming HLA class IA expression level in HLA-expressing Ba/F3 cell lines and ARH77 cells by FACS.
Fig. 2 shows a schematic diagram of cell lines expressing human-mouse chimeric HLA class IA in which one of the HLA class IA domains (α1-α3 domains) is substituted with the corresponding domain of mouse MHC class IA.
Fig. 3 shows a table of the results of epitope analysis on ten antibody clones obtained by immunizing mice with HLA-A/β2 microglobulin (β2M)-coexpressing Ba/F3 cells. The activity of binding to each type of human-mouse chimeric HLA class IA-expressing Ba/F3 cells (MHH, HMH, and HHM) was analyzed by FACS, and (+) denotes confirmed binding and (-) denotes absence of binding. The epitope for each clone was determined from the respective staining patterns.
Fig. 4 shows the result of examining cell death-inducing activity on ARH77 in the presence or absence of a secondary antibody for ten antibody clones obtained by immunizing mice with HLA-A/β2 microglobulin (β2M)-coexpressing Ba/F3 cells.
Fig. 5-1 shows the amino acid sequences of the heavy chain variable regions of 2D7, and the newly obtained C3B3, C17D11, and C11B9.
Fig. 5-2 shows the amino acid sequences of the light chain variable regions of 2D7, and the newly obtained C3B3, C17D11, and C11B9.
Fig. 6 shows a separation chart obtained by purification of the C3B3 minibody using gel filtration chromatography.
Fig. 7 shows a graph indicating the *in vitro* cytotoxic activity of each of Peaks (1)-(3) of the C3B3 minibody separated by gel filtration chromatography, on ARH77.
Fig. 8 shows a graph indicating the *in vitro* cell growth-suppressing activities of the C3B3 diabody (C3B3 DB) and 2D7 diabody (2D7 DB) on ARH77.
Fig. 9 shows graphs indicating the *in vitro* cell growth-suppressing activities of the C3B3 diabody (C3B3 DB) and 2D7 diabody (2D7 DB) on human myeloma cells (ARH77, IM-9, HS-Sultan, MC/CAR).
Fig. 10 shows a graph indicating the survival time of IM-9-transplanted mice when PBS/Tween20 (control), the 2D7 diabody (2D7 DB), or C3B3 diabody (C3B3 DB) was administered.
Fig. 11 shows a graph indicating the amount of serum human IgG in IM-9-transplanted mice on Day 14 after transplantation. PBS/Tween20 (control), the 2D7 diabody (2D7 DB), or C3B3 diabody (C3B3 DB) was administered.
Fig. 12 shows a graph indicating the *in vitro* cytotoxic activity of the C3B3 diabody and 2D7 diabody on human peripheral blood mononuclear cells (PBMCs).
Fig. 13 shows growth-suppressing effects of the C3B3 diabody and 2D7 diabody on human T-cell tumor cells. Growth-suppressing effect of each antibody on Jurkat cells cultured for 3 days are shown.

### Mode for Carrying Out the Invention

The present invention relates to antibodies comprising a heavy chain variable region that comprises CDR 1, 2, and 3 consisting of the amino acid sequences of SEQ ID NOs: 7, 8, and 9. Furthermore, the present invention relates to antibodies comprising a light chain variable region that comprises CDR 1, 2, and 3 consisting of the amino acid sequences of SEQ ID NOs: 10, 11, and 12.

The present inventors used HLA class I as an antigen to obtain new antibodies that have cell death-inducing activity. Among them, three clones (C3B3, C11B9, and C17D11 antibodies) whose epitope is an HLA class I α2 domain, were found to show strong cytotoxic activity when cross-linked with an anti-mouse IgG antibody. Furthermore, by modifying the C3B3 antibody into a low-molecular-weight antibody (diabody) using antibody engineering techniques, the present inventors succeeded in providing an agonistic antibody (C3B3 diabody) that by itself exhibits a stronger anti-tumor effect than a conventional diabody of the 2D7 antibody. The present invention is based on these findings.

The present invention provides antibodies comprising a heavy chain variable region that comprises CDR 1, 2, and 3 consisting of the amino acid sequences of SEQ ID NOs: 7, 8, and 9. The present invention also provides antibodies comprising a light chain variable region that comprises CDR 1, 2, and 3 consisting of the amino acid sequences of SEQ ID NOs: 10, 11, and 12.

The antibodies of the present invention are not particularly limited so long as they comprise a heavy chain variable region that comprises CDR 1, 2, and 3 consisting of the amino acid sequences of SEQ ID NOs: 7, 8, and 9, or a light chain variable region that comprises CDR 1, 2, and 3 consisting of the amino acid sequences of SEQ ID NOs: 10, 11, and 12.

Preferred examples of the antibodies of the present invention include antibodies comprising a heavy chain variable region of any one of (a) to (d) below:
(a) a heavy chain variable region comprising the amino acid sequence of SEQ ID NO: 2;
(b) a heavy chain variable region that comprises an amino acid sequence with one or more amino acid substitutions, deletions, insertions, and/or additions in the amino acid sequence of SEQ ID NO: 2, and that is functionally equivalent to the heavy chain variable region of (a);
(c) a heavy chain variable region comprising an amino acid sequence encoded by a DNA comprising the nucleotide sequence of SEQ ID NO: 1; and
(d) a heavy chain variable region comprising an amino acid sequence encoded by a DNA that hybridizes under stringent conditions with a DNA comprising the nucleotide sequence of SEQ ID NO: 1.
   Alternatively, examples of the antibodies of the present invention include antibodies comprising a light chain variable region of any one of (e) to (h) below:
(e) a light chain variable region comprising the amino acid sequence of SEQ ID NO: 4;
(f) a light chain variable region that comprises an amino acid sequence with one or more amino acid substitutions, deletions, insertions, and/or additions in the amino acid sequence of SEQ ID NO: 4, and that is functionally equivalent to the light chain variable region of (e);
(g) a light chain variable region comprising an amino acid sequence encoded by a DNA comprising the nucleotide sequence of SEQ ID NO: 3; and
(h) a light chain variable region comprising an amino acid sequence encoded by a DNA that hybridizes under stringent conditions with a DNA comprising the nucleotide sequence of SEQ ID NO: 3.

Furthermore, examples of antibodies comprising such heavy chain variable regions and light chain variable regions are antibodies comprising an amino acid sequence of any one of (a) to (d) below:
(a) the amino acid sequence of SEQ ID NO: 6;
(b) an amino acid sequence with one or more amino acid substitutions, deletions, insertions, and/or additions in the amino acid sequence of SEQ ID NO: 6;
(c) an amino acid sequence encoded by a DNA comprising the nucleotide sequence of SEQ ID NO: 5; and
(d) an amino acid sequence encoded by a DNA that hybridizes under stringent conditions with a DNA comprising the nucleotide sequence of SEQ ID NO: 5.

The amino acid sequence of the heavy chain variable region or the light chain variable region may contain substitutions, deletions, additions, and/or insertions. Furthermore, it may also lack portions of heavy chain variable region and/or light chain variable region, or other polypeptides may be added, as long as the binding complex of heavy chain variable regions and light chain variable regions retains its antigen binding activity. Additionally, the variable region may be chimerized or humanized.

Herein, the term "functionally equivalent" means that the antibody of interest has an activity equivalent to the antibody comprising a heavy chain variable region that comprises CDR 1, 2, and 3 consisting of the amino acid sequences of SEQ ID NOs: 7, 8, and 9, or a light chain variable region that comprises CDR 1, 2, and 3 consisting of the amino acid sequences of SEQ ID NOs: 10, 11, and 12 (for example, HLA-A binding activity, cell death-inducing activity, or such).

Methods for preparing polypeptides functionally equivalent to a certain polypeptide are well known to those skilled in the art, and include methods of introducing mutations into polypeptides. For example, one skilled in the art can prepare an antibody functionally equivalent to an antibody of the present invention by introducing appropriate mutations into the antibody using site-directed mutagenesis(Hashimoto-Gotoh, T. et al. (1995) Gene 152, 271-275; Zoller, MJ, and Smith, M.(1983) Methods Enzymol. 100, 468-500; Kramer, W. et al. (1984) Nucleic Acids Res. 12, 9441-9456; Kramer W, and Fritz HJ(1987) Methods. Enzymol. 154, 350-367; Kunkel, TA (1985) Proc Natl. Acad. Sci. USA. 82,488-492; Kunkel (1988) Methods Enzymol. 85, 2763-2766). Amino acid mutations may also occur naturally. Therefore, the antibodies of the present invention also comprise antibodies functionally equivalent to the antibodies of the present invention, wherein the antibodies comprises amino acid sequences with one or more amino acid mutations to the amino acid sequences of the present invention's antibodies.

The number of amino acids that are mutated is not particularly limited, but is generally 30 amino acids or less, preferably 15 amino acids or less, and more preferably 5 amino acids or less (for example, 3 amino acids or less). Preferably, the mutated amino acids conserve the properties of the amino acid side chain from the amino acids that were mutated. Examples of amino acid side chain properties include: hydrophobic amino acids (A, I, L, M, F, P, W, Y, and V), hydrophilic amino acids (R, D, N, C, E, Q, G, H, K, S, and T), amino acids comprising the following side chains: aliphatic side chains (G, A, V, L, I, and P); hydroxyl-containing side chains (S, T, and Y); sulfur-containing side chains (C and M); carboxylic acid- and amide-containing side chains (D, N, E, and Q); basic side chains (R, K, and H); and aromatic ring-containing side chains (H, F, Y, and W) (amino acids are represented by one-letter codes in parentheses). Polypeptides comprising a modified amino acid sequence, in which one or more amino acid residues is deleted, added, and/or substituted with other amino acids, are known to retain their original biological activities (Mark, D. F. et al., Proc. Natl. Acad. Sci. USA (1984) 81, 5662-5666; Zoller, M. J. & Smith, M. Nucleic Acids Research (1982) 10, 6487-6500; Wang, A. et al., Science 224, 1431-1433; Dalbadie-McFarland, G et al., Proc. Natl. Acad. Sci. (1982) USA 79, 6409-6413).

The antibodies of the present invention also include, antibodies in which several amino acid residues have been added to an amino acid sequence of an antibody of the present invention. Fusion proteins in which such antibodies are fused together with other peptides or proteins are also included in the present invention. A fusion protein can be prepared by ligating a polynucleotide encoding an antibody of the present invention and a polynucleotide encoding another peptide or polypeptide such that the reading frames match, inserting this into an expression vector, and expressing the fusion construct in a host. Techniques known to those skilled in the art are available for this purpose. The peptides or polypeptides to be fused with an antibody of the present invention include, for example, FLAG (Hopp, T.P. et al., Biotechnology (1988) 6, 1204-1210), 6x His consisting of six His (histidine) residues, 10x His, Influenza hemagglutinin (HA), human c-myc fragment, VSV-GP fragment, p18HIV fragment, T7-tag, HSV-tag, E-tag, SV40T antigen fragment, lck tag, α-tubulin fragment, B-tag, Protein C fragment, and such. Examples of other polypeptides to be fused to the antibodies of the present invention include, GST (glutathione-S-transferase), HA (Influenza hemagglutinin), immunoglobulin constant region, β-galactosidase, MBP (maltose-binding protein), and such. Commercially available polynucleotides encoding these peptides or polypeptides can be fused with polynucleotides encoding the antibodies of the present invention. The fusion polypeptide can be prepared by expressing the fusion construct.

Furthermore, the present invention also provides antibodies that bind to the same epitopes as the epitopes to which the antibodies disclosed in the application of the present invention bind. More specifically, the present invention relates to antibodies that recognize the same epitopes as the epitopes recognized by the antibodies of the present invention, and uses thereof. Such an antibody can be obtained, for example, by the following methods.

Whether a test antibody binds to the same epitope as the epitope to which a certain antibody binds, that is, whether an epitope is shared between a test antibody and a certain antibody can be confirmed by competition between the two antibodies for the same epitope. In the present invention, competition between antibodies can be detected by FACS, cross-blocking assay, or such. In FACS, a monoclonal antibody is first bound to cells that express HLA-IA on their surface, and the fluorescence signal is measured. Next, after a candidate competing antibody is reacted with the cells, an antibody of the present invention is reacted with the same cells, and this is analyzed by FACS in a similar manner. Alternatively, a monoclonal antibody of the present invention and a test competing antibody can be reacted with the same cells at the same time. If the FACS analysis pattern of the antibody of the present invention changes when the competing antibody is reacted with the cells, it can be confirmed that the competing antibody and the antibody of the present invention recognize the same epitope.

In addition, competitive ELISA assay, for example, is a preferred cross-blocking assay. More specifically, in a cross-blocking assay, HLA-IA-expressing cells are fixed to the wells of a microtiter plate. After pre-incubation with or without a candidate competing antibody, a monoclonal antibody of the present invention is added. The amount of the antibody of the present invention bound to the HLA-IA expressing cells in the wells is inversely correlated to the binding ability of the candidate competing antibody (test antibody) that competes for binding to the same epitope. More specifically, the greater the affinity of the test antibody for the same epitope, the less amount of the antibody of the present invention will be bound to the wells fixed with the HLA-IA protein-expressing cells. In other words, the greater the affinity of a test antibody to the same epitope, the greater amount of the test antibody will be bound to the wells fixed with the HLA-IA protein-expressing cells.

The amount of antibody that binds to the wells can be measured easily by labeling the antibody in advance. For example, a biotin-labeled antibody can be measured using an avidin-peroxidase conjugate and suitable substrate. Cross-blocking assays using enzyme labels such as peroxidase are called competitive ELISA assay, in particular. The antibody can be labeled with other detectable or measurable labeling substances. More specifically, radiolabels or fluorescent labels are known.

Furthermore, when the test antibody comprises a constant region derived from a different species than that of the antibody of the present invention, any antibody bound to the wells can be measured using a labeled antibody that specifically recognizes the constant region derived from any of the species. Even if the antibody is derived from the same species, if the class is different, antibodies bound to the wells can be measured using an antibody that specifically distinguishes each class.

A candidate competing antibody is considered to be an antibody that binds substantially to the same epitope, or competes for binding to the same epitope as the antibody of the present invention, if the candidate antibody can block binding of the monoclonal antibody of the present invention by at least 20%, preferably at least 20-50%, and more preferably at least 50% when compared with the binding activity obtained in the control experiment performed in the absence of the candidate competing antibody.

The antibody that binds to the same epitope as the epitope to which the antibody of the present invention binds is, for example, the antibody of [8] or [9] mentioned above.

As described above, the antibody of [8] or [9] mentioned above includes not only monovalent antibodies but also polyvalent antibodies. The polyvalent antibodies of the present invention include polyvalent antibodies having the same antigen binding sites, and polyvalent antibodies having partially or completely different antigen binding sites.

As described below, the antibodies of the present invention may differ in amino acid sequence, molecular weight, and isoelectric point, and may also be different in the presence or absence of sugar chains and conformation, depending on the cell or host producing the antibody or purification method. However, as long as the obtained antibody is functionally equivalent to an antibody of the present invention, it is included in the present invention. For example, when an antibody of the present invention is expressed in a prokaryotic cell such as E. *coli,* a methionine residue is added to the N terminus of the amino acid sequence of the original antibody. The antibodies of the present invention will also include such antibodies.

The antibodies of the present invention may be conjugated antibodies that are bound to various molecules, including, for example, polyethylene glycol (PEG), radioactive substances, and toxins. Such conjugate antibodies can be obtained by chemically modifying the obtained antibodies. Methods for antibody modification are already established in this field (see for example, US5057313, and US5156840). Accordingly, the term "antibody" as used herein includes such conjugate antibodies.

Mouse antibodies, rat antibodies, rabbit antibodies, sheep antibodies, camel antibodies, chimeric antibodies, humanized antibodies, human antibodies, and such may be used for the antibodies of the present invention as necessary. Furthermore, low-molecular-weight antibodies and such may be used as the antibodies of the present invention.

For the antibodies of the present invention, genetically modified antibodies produced by incorporating an antibody gene into a suitable vector and introducing this vector into a host using genetic engineering techniques (for example, see Carl, A. K. Borrebaeck, James, W. Larrick, THERAPEUTIC MONOCLONAL ANTIBODIES, Published in the United Kingdom by MACMILLAN PUBLISHERS LTD, 1990) can be used. More specifically, when DNAs encoding heavy chain variable regions that comprise CDR 1, 2, and 3 consisting of the amino acid sequences of SEQ ID NOs: 7, 8, and 9, or light chain variable regions that comprise CDR 1, 2, and 3 consisting of the amino acid sequences of SEQ ID NOs: 10, 11, and 12 are obtained, they are linked to a DNA encoding a desired antibody constant region (C region), and this is then incorporated into an expression vector. Alternatively, a DNA encoding an antibody variable region can be incorporated into an expression vector that comprises a DNA of an antibody constant region. The DNA is incorporated into the expression vector such that it is expressed under the control of an expression regulatory region (for example, enhancer or promoter). The antibody can then be expressed by transforming host cells using this expression vector.

The present invention also provides polynucleotides encoding the antibodies of the present invention, or polynucleotides that hybridize under stringent conditions to the polynucleotides of the present invention and encode antibodies having an activity equivalent to that of the antibodies of this invention. The polynucleotides of the present invention are polymers comprising multiple nucleic bases or base pairs of deoxyribonucleic acids (DNA) or ribonucleic acids (RNA), and are not particularly limited, as long as they encode the antibodies of the present invention. Polynucleotides of the present invention may also contain non-natural nucleotides. The polynucleotides of the present invention can be used to express antibodies using genetic engineering techniques. Furthermore, they can be used as probes in the screening of antibodies functionally equivalent to the antibodies of the present invention. Specifically, DNAs that hybridize under stringent conditions to the polynucleotides encoding the antibodies of the present invention, and encode antibodies having an activity equivalent to that of the antibodies of the present invention, can be obtained by techniques such as hybridization and gene amplification (for example, PCR), using a polynucleotide of the present invention or a portion thereof as a probe. Such DNAs are included in the polynucleotides of the present invention. Hybridization techniques are well known to those skilled in the art (Sambrook, J. et al., Molecular Cloning 2nd ed., 9.47-9.58, Cold Spring Harbor Lab. press, 1989). Conditions for hybridization may include ,for example, those with low stringency. Examples of conditions of low stringency include post-hybridization washing in 0.1x SSC and 0.1% SDS at 42°C, and preferably in 0.1x SSC and 0.1% SDS at 50°C. More preferable hybridization conditions include those of high stringency. Highly stringent conditions include, for example, washing in 5x SSC and 0.1% SDS at 65°C. In these conditions, the higher the temperature, the more it can be expected that a polynucleotide with a high homology would be obtained. However, several factors such as temperature and salt concentration can influence hybridization stringency, and those skilled in the art can suitably select these factors to accomplish similar stringencies.

An antibody encoded by a polynucleotide obtained by a hybridization and gene amplification technique, and is functionally equivalent to an antibody of the present invention, generally has high homology to the amino acid sequence of the antibody of this invention. The antibodies of the present invention include antibodies that are functionally equivalent and have high amino acid sequence homology to the antibodies of the present invention. The term "high homology" generally means identity at the amino acid level of at least 50% or higher, preferably 75% or higher, more preferably 85% or higher, still more preferably 95% or higher. Polypeptide homology can be determined by the algorithm described in Wilbur, W. J. and Lipman, D. J. Proc. Natl. Acad. Sci. USA 80, 726-730 (1983).

Preferred examples of polynucleotides encoding the antibodies of the present invention include polynucleotides of (a) and (b):
(a) a polynucleotide comprising the nucleotide sequence of SEQ ID NOs: 1, 3, or 5; or
(b) a polynucleotide that hybridizes under stringent conditions with the polynucleotide of (a), and encodes an antibody having an activity equivalent to the antibody of the present invention.

Appropriate combinations of host and expression vector can be used when an antibody is produced by first isolating the antibody gene and then introducing it into a suitable host.

The present invention provides vectors comprising the above-mentioned polynucleotides. The vectors include, for example, M13 vectors, pUC vectors, pBR322, pBluescript, and pCR-Script. In addition to the above vectors, for example, pGEM-T, pDIRECT, and pT7 can also be used for the subcloning and excision of cDNAs. When using vectors to produce the antibodies of this invention, expression vectors are particularly useful. When an expression vector is expressed in *E. coli,* for example, it should have the above characteristics in order to be amplified in *E*. *coli.* Additionally, when *E*. *coli* such as JM109, DH5 α, HB101, or XL1-Blue are used as the host cell, the vector necessarily has a promoter, for example, a lacZ promoter (Ward et al. (1989) Nature 341:544-546; (1992) FASEB J. 6:2422-2427), araB promoter (Better et al. (1988) Science 240:1041-1043), or T7 promoter, to allow efficient expression of the desired gene in *E*. *coli.* Other examples of the vectors include pGEX-5X-1 (Pharmacia), "QIAexpress system" (QIAGEN), pEGFP, and pET (where BL21, a strain expressing T7 RNA polymerase, is preferably used as the host).

Furthermore, the vector may comprise a signal sequence for polypeptide secretion. When producing proteins into the periplasm of *E. coli,* the pelB signal sequence (Lei, S. P. et al. J. Bacteriol. 169:4379 (1987)) may be used as a signal sequence for protein secretion. For example, calcium chloride methods or electroporation methods may be used to introduce the vector into a host cell.

In addition to expression vectors for *E*. *coli,* expression vectors derived from mammals (e.g., pcDNA3 (Invitrogen), pEGF-BOS (Nucleic Acids Res. (1990) 18(17):5322), pEF, pCDM8), insect cells (e.g., "Bac-to-BAC baculovirus expression system" (GIBCO-BRL), pBacPAK8), plants (e.g., pMH1, pMH2), animal viruses (e.g., pHSV, pMV, pAdexLcw), retroviruses (e.g., pZIPneo), yeasts (e.g., "Pichia Expression Kit" (Invitrogen), pNV11, SP-Q01), and *Bacillus subtilis* (e.g., pPL608, pKTH50) may also be used as vectors for producing the polypeptides of the present invention.

In order to express proteins in animal cells, such as CHO, COS, and NIH3T3 cells, the vector necessarily has a promoter necessary for expression in such cells, for example, an SV40 promoter (Mulligan et al. (1979) Nature 277:108), MMLV-LTR promoter, EF1αpromoter (Mizushima et al. (1990) Nucleic Acids Res. 18:5322), CMV promoter, etc. It is even more preferable that the vector also carries a marker gene for selecting transformants (for example, a drug-resistance gene enabling selection by a drug such as neomycin and G418). Examples of vectors with such characteristics include pMAM, pDR2, pBK-RSV, pBK-CMV, pOPRSV, pOP13, and such.

In addition, to stably express a gene and amplify the gene copy number in cells, CHO cells having a defective nucleic acid synthesis pathway can be introduced with a vector containing a DHFR gene (for example, pCHOI) to compensate for the defect, and the copy number may be amplified using methotrexate (MTX). Alternatively, a COS cell, which carries an SV40 T antigen-expressing gene on its chromosome, can be transformed with a vector containing the SV40 replication origin (for example, pcD) for transient gene expression. The replication origin may be derived from polyoma viruses, adenoviruses, bovine papilloma viruses (BPV), and such. Furthermore, to increase the gene copy number in host cells, the expression vector may contain, as a selection marker, an aminoglycoside transferase (APH) gene, thymidine kinase (TK) gene, *E*. *coli* xanthine guanine phosphoribosyl transferase (Ecogpt) gene, dihydrofolate reductase (dhfr) gene, and such.

Methods for expressing polynucleotides of this invention in animal bodies include methods of incorporating the polynucleotides of this invention into appropriate vectors and introducing them into living bodies by, for example, a retrovirus method, liposome method, cationic liposome method, or adenovirus method. The vectors that are used include adenovirus vectors (for example, pAdexlcw), and retrovirus vectors (for example, pZIPneo), but are not limited thereto. General genetic manipulations such as inserting the polynucleotides of this invention into vectors can be performed according to conventional methods (Molecular Cloning, 5.61-5.63). Administration to living bodies can be carried out by *ex vivo* or *in vivo* methods.

Furthermore, the present invention provides host cells into which a vector of this invention is introduced. The host cells are not particularly limited; for example, *E*. *coli* and various animal cells are available for this purpose. The host cells of this invention may be used, for example, as production systems to produce and express the antibodies of the present invention. *In vitro* and *in vivo* production systems are available for polypeptide production systems. Production systems that use eukaryotic cells or prokaryotic cells are examples of *in vitro* production systems.

Eukaryotic cells that can be used include, for example, animal cells, plant cells, and fungal cells. Known animal cells include: mammalian cells, for example, CHO (J. Exp. Med. (1995)108, 945), COS, NIH3T3, myeloma, BHK (baby hamster kidney), HeLa, Vero, amphibian cells such as *Xenopus laevis* oocytes (Valle, et al. (1981) Nature 291, 358-340), or insect cells (e.g., Sf9, Sf21, and Tn5). CHO cells in which the DHFR gene has been deleted, such as dhfr-CHO (Proc. Natl. Acad. Sci. USA (1980) 77, 4216-4220) and CHO K-1 (Proc. Natl. Acad. Sci. USA (1968) 60, 1275), are particularly preferable for use as CHO cells. Of the animal cells, CHO cells are particularly favorable for large-scale expression. Vectors can be introduced into a host cell by, for example, calcium phosphate method, DEAE-dextran method, method using cationic ribosome DOTAP (Boehringer-Mannheim), electroporation methods, lipofection methods, etc.

Plant cells including, for example, Nicotiana tabacum-derived cells are known as polypeptide production systems. Calluses may be cultured from these cells. Known fungal cells include yeast cells, for example, the genus Saccharomyces, such as *Saccharomyces cerevisiae*; and filamentous fungi, for example, the genus Aspergillus such as *Aspergillus niger*.

Bacterial cells can be used in prokaryotic production systems. Examples of bacterial cells include *E*. *coli* (for example, JM109, DH5α, HB101 and such); and *Bacillus subtilis*.

Antibodies can be obtained by transforming the cells with a polynucleotide of interest, then culturing these transformants *in vitro.* Transformants can be cultured using known methods. For example, DMEM, MEM, RPMI 1640, or IMDM may be used as the culture medium for animal cells, and may be used with or without serum supplements such as fetal calf serum (FCS). Serum-free cultures are also acceptable. The preferred pH is about 6 to 8 over the course of culturing. Incubation is typically carried out at a temperature of about 30 to 40°C for about 15 to 200 hours. Medium is exchanged, aerated, or agitated, as necessary.

On the other hand, production systems using animal or plant hosts may be used as systems for producing polypeptides *in vivo*. For example, a polynucleotide of interest may be introduced into an animal or plant, and the polypeptide produced in the body of the animal or plant is then recovered. The "hosts" of the present invention include such animals and plants.

When using animals, there are production systems using mammals or insects. Mammals such as goats, pigs, sheep, mice, and cattle may be used (Vicki Glaser SPECTRUM Biotechnology Applications (1993)). Alternatively, the mammals may be transgenic animals.

For example, a polynucleotide of interest may be prepared as a fusion gene with a gene encoding a polypeptide specifically produced in milk, such as the goat β-casein gene. Polynucleotide fragments containing the fusion gene are injected into goat embryos, which are then introduced back to female goats. The desired antibody can then be obtained from milk produced by the transgenic goats, which are born from the goats that received the embryos, or from their offspring. Appropriate hormones may be administered to increase the volume of milk containing the polypeptide produced by the transgenic goats (Ebert, K.M. et al., Bio/Technology 12, 699-702 (1994)).

Insects, such as silkworms, may also be used. Baculoviruses carrying a polynucleotide of interest can be used to infect silkworms, and the antibody of interest can be obtained from their body fluids (Susumu, M. et al., Nature 315, 592-594 (1985)).

When using plants, tobacco can be used, for example. When tobacco is used, a polynucleotide of interest may be inserted into a plant expression vector, for example, pMON 530, and then the vector may be introduced into a bacterium such as *Agrobacterium tumefaciens*. The bacteria are then used to infect tobacco, such as *Nicotiana tabacum*, and the desired polypeptides are recovered from the leaves (Julian K.-C. Ma et al., Eur. J. Immunol. 24, 131-138 (1994)).

The resulting antibodies of this invention may be isolated from the inside or outside (such as the medium) of host cells, and purified as substantially pure and homogenous antibodies. Any standard method for isolating and purifying antibodies may be used, and methods are not limited to any specific method. Antibodies may be isolated and purified by selecting an appropriate combination of, for example, chromatographic columns, filtration, ultrafiltration, salting out, solvent precipitation, solvent extraction, distillation, immunoprecipitation, SDS-polyacrylamide gel electrophoresis, isoelectric focusing, dialysis, recrystallization, and others.

Chromatography includes, for example, affinity chromatography, ion exchange chromatography, hydrophobic chromatography, gel filtration, reverse-phase chromatography, adsorption chromatography and the like (Strategies for Protein Purification and Characterization: A Laboratory Course Manual. Ed Daniel R. Marshak et al., Cold Spring Harbor Laboratory Press, 1996). These chromatographies can be carried out using liquid phase chromatographies such as HPLC and FPLC. Examples of columns used in affinity chromatography include protein A column and protein G column. Columns using protein A column include, for example, Hyper D, POROS, Sepharose F. F. (Pharmacia) and the like. The present invention also includes antibodies that are highly purified using these purification methods.

In the present invention, the antigen-binding activity of the prepared antibodies (Antibodies A Laboratory Manual. Ed Harlow, David Lane, Cold Spring Harbor Laboratory, 1988) can be measured using well known techniques. For example, ELISA (enzyme linked immunosorbent assay), EIA (enzyme immunoassay), RIA (radioimmunoassay), or fluoroimmunoassay may be used.

The present invention provides antibody production methods comprising the steps of producing an above-mentioned polynucleotide, producing a vector comprising the polynucleotide, introducing the vector into host cells, and culturing the host cells.

Alternatively, in the present invention, artificially modified genetically-recombinant antibodies, such as chimeric and humanized antibodies, may be used to reduce heterologous antigenicity against human and such. These modified antibodies can be produced using known methods. A chimeric antibody is an antibody comprising the heavy and light chain variable regions of an antibody from a non-human mammal such as mouse, and the heavy and light chain constant regions of a human antibody. The chimeric antibody can be produced by linking a DNA encoding a mouse antibody variable region with a DNA encoding a human antibody constant region, incorporating this into an expression vector, and then introducing the vector into a host.

Humanized antibodies are also referred to as "reshaped human antibodies". Such humanized antibodies are obtained by grafting the complementarity determining region (CDR) of an antibody derived from a non-human mammal, for example, a mouse, to the CDR of a human antibody, and such general gene recombination procedures are also known. Specifically, a DNA sequence designed to link a murine antibody CDR to the framework region (FR) of a human antibody is synthesized by PCR, using several oligonucleotides produced to contain overlapping portions in the terminal regions. The obtained DNA is linked to a DNA encoding a human antibody constant region, and this is then integrated into an expression vector, and the antibody is produced by introducing this vector into a host (see European Patent Application EP 239400, and International Patent Application WO 96/02576). The human antibody FR to be linked via CDR is selected so that the CDR forms a favorable antigen-binding site. In order for the CDR of the reshaped human antibody to form a suitable antigen-binding site, the amino acids in the framework region of the antibody variable region may be substituted as necessary (Sato, K. et al., 1993, Cancer Res. 53, 851-856).

Methods for obtaining human antibodies are also known. For example, human lymphocytes can be sensitized *in vitro* with a desired antigen, or with cells expressing a desired antigen, and the sensitized lymphocytes can be fused with human myeloma cells such as U266, to obtain the desired human antibody with antigen-binding activity (see Japanese Patent Application Kokoku Publication No. (JP-B) Hei 1-59878 (examined, approved Japanese patent application published for opposition)). Further, a desired human antibody can be obtained by using a desired antigen to immunize transgenic animals that have a full repertoire of human antibody genes (see International Patent Application WO 93/12227, WO 92/03918, WO 94/02602, WO 94/25585, WO 96/34096, and WO 96/33735). Furthermore, techniques for obtaining human antibodies by panning using a human antibody library are also known. For example, variable regions of human antibodies can be expressed as single-chain antibodies (scFvs) on the surface of phages using phage display methods, and phages that bind to antigens can be selected. DNA sequences encoding the variable regions of human antibodies that bind to the antigens can be determined by analyzing the genes of the selected phages. By revealing the DNA sequences of the scFvs that bind to the antigens, appropriate expression vectors carrying the sequences can be produced to yield human antibodies. These methods are already known, and the following publications can be referred to: WO 92/01047, WO 92/20791, WO 93/06213, WO 93/11236, WO 93/19172, WO 95/01438, and WO 95/15388.

The antibodies of the present invention are preferably antibodies that recognize human leukocyte antigens (HLA). Antibodies of the present invention which recognize human leukocyte antigens (HLA) are useful because they have enhanced activity. Herein, "activity" refers to a biological action that arises as a result of antigen-antibody binding. Specific examples of the biological action include cell death induction, apoptosis induction, cell growth suppression, cell differentiation suppression, cell division suppression, cell growth induction, cell differentiation induction, cell division induction, and cell cycle regulation and the like. Cell death induction and cell growth suppression are preferred.

Cells that become a target of the above-mentioned actions, such as cell death induction and cell growth suppression, are not particularly limited, though hematopoietic cells and non-adherent cells are preferred. Specific examples of hematopoietic cells include lymphocytes (B cells, T cells), neutrophils, eosinophils, basophils, monocytes (preferably activated peripheral blood mononuclear cells (PBMC)), and hematopoietic tumor cells (myeloma cells, lymphoma cells, and leukemia cells), and are preferably lymphocytes (B cells, T cells, activated B cells, and activated T cells), particularly activated B cells or activated T cells, and most preferably hematopoietic tumor cells. "Non-adherent cells" refers to cells that, when cultured, grow in a non-adherent state without adhering to the surface of culturing vessels such as glass or plastic. Preferred examples of non-adherent cells in the present invention include Jurkat cells and ARH77 cells. On the other hand, "adherent cells" refers to cells that, when cultured, adhere to the surface of culturing vessels such as glass or plastic.

Generally, a full length anti-HLA antibody may be cross-linked with an anti-IgG antibody or such to exhibit enhanced cell death-inducing activity, and cross-linking can be carried out by those skilled in the art using known methods.

Whether or not the antibodies of the present invention will induce cell death in non-adherent cells can be determined by observing induction of cell death in Jurkat cells or ARH77 cells. Whether or not the antibodies will induce cell death in adherent cells can be determined by observing induction of cell death in HeLa cells (WO2004/033499).

In the present invention, administration of the above-mentioned HLA-recognizing antibody can treat or prevent diseases such as tumors including hematopoietic tumors (specific examples include leukemia; myelodysplastic syndrome; malignant lymphoma; chronic myelogenic leukemia; plasmacytic disorders such as myeloma, multiple myeloma, and macroglobulinemia; and myeloproliferative diseases such as polycythemia vera, essential thrombocythemia, and idiopathic myelofibrosis; and such), and autoimmune diseases (specific examples include rheumatism, autoimmune hepatitis, autoimmune thyroiditis, autoimmune bullosis, autoimmune adrenocortical disease, autoimmune hemolytic anemia, autoimmune thrombycytopenic purpura, autoimmune atrophic gastritis, autoimmune neutropenia, autoimmune orchitis, autoimmune encephalomyelitis, autoimmune receptor disease, autoimmune infertility, Crohn's disease, systemic lupus erythematosus, multiple sclerosis, Basedow's disease, juvenile diabetes, Addison's disease, myasthenia gravis, lens-induced uveitis, psoriasis, and Behchet's disease). Furthermore, the excellent stability of the present invention's antibodies *in vivo* would be particularly efficacious when administered to living subjects.

In the present invention, HLA refers to human leukocyte antigen. HLA molecules are categorized into class I and class II. Known examples of class I are HLA-A, B, C, E, F, G, H, J, and such; and known examples of class II are HLA-DR, DQ, DP, and such. The antigens recognized by the antibodies of the present invention are not particularly limited, so long as they are HLA molecules, and are preferably molecules classified as class I, and more preferably HLA-IA.

Antibodies of the present invention may be low-molecular-weight antibodies. In the present invention, low-molecular-weight antibodies include antibody fragments in which part of a whole antibody (for example, whole IgG) is missing, and are not particularly limited so long as they have antigen-binding ability. Antibody fragments of the present invention are not particularly limited so long as they are part of a whole antibody. However, fragments comprising heavy chain variable regions (VH) or light chain variable regions (VL) are preferred, and fragments comprising both VH and VL are particularly preferred. Specific examples of antibody fragments include Fab, Fab', F(ab')2, Fv, scFv (single chain Fv), sc(Fv)₂and such, but are preferably diabodies (Huston, J. S. et al., Proc. Natl. Acad. Sci. U.S.A. (1988) 85, 5879-5883; Plickthun "The Pharmacology of Monoclonal Antibodies" Viol.113, Resenburg and Moore ed., Springer Verlag, New York, pp.269-315, (1994)). Such antibody fragments can be obtained by treating an antibody with enzymes such as papain or pepsin to produce antibody fragments, or by constructing genes encoding such antibody fragments, introducing them into an expression vector, and then expressing this in an appropriate host cell (see for example, Co, M. S. et al., J. Immunol. (1994) 152, 2968-2976; Better, M. and Horwitz, A. H., Methods Enzymol. (1989) 178, 476-496; Pluckthun, A. and Skerra, A., Methods Enzymol. (1989) 178, 497-515; Lamoyi, E., Methods Enzymol. (1986) 121, 652-663; Rousseaux, J. et al., Methods Enzymol. (1986) 121, 663-669; Bird, R. E. and Walker, B. W., Trends Biotechnol. (1991) 9, 132-137).

The molecular weight of the low-molecular-weight antibody of the present invention is preferably smaller than that of the whole antibody, but multimers such as dimers, trimers, and tetramers may be formed, and the molecular weight can be larger than the molecular weight of the whole antibody.

Low-molecular-weight antibodies of the present invention are preferably antibodies comprising two or more VH and two or more VL of an antibody, and these variable regions are linked directly or indirectly through linkers or such. The linkages may be covalent bonds, non-covalent bonds, or both covalent and non-covalent bonds. A more preferable low-molecular-weight antibody is an antibody comprising two or more VH-VL pairs formed by linking VH and VL with a non-covalent bond. In this case, a low-molecular-weight antibody having a shorter distance between one VH-VL pair and the other VH-VL pair than the distance in the whole antibody is preferred.

In the present invention, scFv is obtained by ligating an antibody H chain V region with an antibody L chain V region. In this scFv, the H chain V region and L chain V region are ligated via a linker, preferably via a peptide linker (Huston, J. S. et al., Proc. Natl. Acad. Sci. U.S.A. (1988) 85, 5879-5883). The H-chain V-region and L-chain V-region in scFv may be derived from any of the antibodies described herein. For example, any single-chain peptides consisting of 12 to 19 amino acid residues may be used as a peptide linker for ligating the V regions.

DNAs encoding scFv can be obtained by using as a template, DNAs encoding the antibody H chain or H chain V region and the antibody L chain or L chain V region mentioned above, and among those sequences, amplifying a DNA portion that encodes the desired amino acid sequence by PCR using a primer pair that defines its two ends; and then carrying out a subsequent amplification using a combination of a DNA encoding the peptide linker portion, and the primer pair that defines both ends of the linker DNA to be ligated to the H chain and the L chain, respectively.

Once a DNA encoding scFv is constructed, an expression vector containing the DNA, and a host transformed with the expression vector can be obtained according to conventional methods. Furthermore, scFvs can be obtained using these hosts according to conventional methods.

These antibody fragments can be produced in hosts by obtaining their genes and expressing them in a manner similar to that described above. These antibody fragments are included in the "antibodies" of the present invention.

Low-molecular-weight antibodies that are particularly preferred in the present invention are diabodies. Diabodies are dimers formed by linking two fragments (such as scFvs; hereinafter referred to as diabody-constituting fragments), in which a variable region is linked to another variable region via a linker or such. Ordinarily, diabodies comprise two VLs and two VHs (P. Holliger et al., Proc. Natl. Acad. Sci. USA, 90, 6444-6448 (1993); EP 404097; WO 93/11161; Johnson et al., Method in Enzymology, 203, 88-98, (1991); Holliger et al., Protein Engineering, 9, 299-305, (1996); Perisic et al., Structure, 2, 1217-1226, (1994); John et al., Protein Engineering, 12(7), 597-604, (1999); Holliger et al,. Proc. Natl. Acad. Sci. USA., 90, 6444-6448, (1993); Atwell et al., Mol. Immunol. 33, 1301-1312, (1996)). Bonds between diabody-constituting fragments may be non-covalent or covalent bonds, but are preferably non-covalent bonds.

Alternatively, diabody-constituting fragments may be linked to each other by a linker and such to form a single-chain diabody (sc diabody). In such case, linking diabody-constituting fragments using a long linker of about 20 amino acids allows diabody-constituting fragments on the same chain to form a dimer with each other via non-covalent bonds.

Diabody-constituting fragments include those with linked VL-VH, VL-VL, and VH-VH, and are preferably those with linked VH-VL. In diabody-constituting fragments, the linker used to link a variable region to a variable region is not particularly limited, but is preferably a linker short enough to prevent non-covalent bonding between variable regions in the same fragment. The length of such a linker can be suitably determined by those skilled in the art, and is ordinarily 2 to 14 amino acids, preferably 3 to 9 amino acids, and most preferably 4 to 6 amino acids. In this case, linkers between VL and VH encoded on a same fragment are short, and thus VL and VH on a same strand do not form a non-covalent bond and therefore a single-chain V region fragment will not be formed. Rather, a fragment forms a dimer with another fragment via non-covalent bonding. Furthermore, according to the same principle in diabody construction, three or more diabody-constituting fragments may be linked to form multimeric antibodies such as trimers and tetramers.

Examples of the diabodies of this invention include, but are not limited to, a diabody comprising the amino acid sequence of SEQ ID NO: 6; a diabody that is functionally equivalent to a diabody comprising the sequence of SEQ ID NO: 6 and has an amino acid sequence with one or more amino acid mutations (substitutions, deletions, insertions, and/or additions) in the amino acid sequence of SEQ ID NO: 6; a diabody comprising the amino acid sequences of the CDRs (or variable regions) of SEQ ID NO: 2 and SEQ ID NO: 4; and a diabody that is functionally equivalent to a diabody comprising the amino acid sequences of the CDRs (or variable regions) of SEQ ID NO: 2 and SEQ ID NO: 4, and has an amino acid sequence with amino acid mutations (substitutions, deletions, insertions, and/or additions) in the amino acid sequences of the CDRs (or variable regions) of SEQ ID NO: 2 and SEQ ID NO: 4.

Herein, "functionally equivalent" means that the diabody of interest has an equivalent activity to that of a diabody comprising the sequence of SEQ ID NO: 6, or that of a diabody comprising the sequences of the CDRs (or variable regions) of SEQ ID NO: 2 and SEQ ID NO: 4 (for example, HLA-A binding activity, and cell death-inducing activity).

The number of mutated amino acids is not particularly limited, but is usually 30 amino acids or less, preferably 15 amino acids or less, and more preferably five amino acids or less (for example, three amino acids or less).

Furthermore, a diabody comprising the amino acid sequence of SEQ ID NO: 6, or a diabody comprising the sequences of the CDRs (or variable regions) of SEQ ID NO: 2 and SEQ ID NO: 4 may be humanized or chimerized to reduce heterologous antigenicity against human.

In the amino acid sequence of SEQ ID NO: 2, amino acids 1 to 125 correspond to the variable region, amino acids 31 to 35 correspond to CDR1 (SEQ ID NO: 7), amino acids 50 to 66 correspond to CDR2 (SEQ ID NO: 8), and amino acids 99 to 114 correspond to CDR3 (SEQ ID NO: 9). In the amino acid sequence of SEQ ID NO: 4, amino acids 1 to 107 correspond to the variable region, amino acids 24 to 34 correspond to CDR1 (SEQ ID NO: 10), amino acids 50 to 56 correspond to CDR2 (SEQ ID NO: 11), and amino acids 89 to 97 correspond to CDR3 (SEQ ID NO: 12).

In the present invention, the HLA-recognizing low-molecular-weight antibodies specifically bind to HLA, and are not particularly limited, so long as they have biological activities. The low-molecular-weight antibodies of the present invention can be prepared by methods well known to those skilled in the art. For example, as described in the Examples, the antibodies can be prepared based on the sequence of an HLA-recognizing antibody (particularly, sequences of the variable regions and CDRs), using genetic engineering techniques known to those skilled in the art.

For the sequence of the HLA-recognizing antibody, a well-known antibody sequence can be used. Alternatively, an anti-HLA antibody can be prepared by a method well known to those skilled in the art using HLA as the antigen, and then the sequence of this antibody can be obtained and then used. Specifically, for example, this can be performed as follows: HLA protein or its fragment is used as a sensitizing antigen to perform immunization according to conventional immunization methods, the obtained immunocytes are fused with known parent cells according to conventional cell fusion methods, and monoclonal antibody-producing cells (hybridomas) are then screened by general screening methods. Antigens can be prepared by known methods, such as methods using baculoviruses (WO98/46777 and such). Hybridomas can be prepared according to the method of Milstein *et al.* (Kohler, G and Milstein, C., Methods Enzymol. (1981) 73:3-46), for example. When an antigen has low immunogenicity, immunization can be performed by binding the antigen to an immunogenic macromolecule such as albumin. Then, cDNAs of the antibody variable region (V region) are synthesized from the mRNAs of the hybridomas using reverse transcriptase, and the sequences of the obtained cDNAs can be determined by known methods.

Antibodies that recognize HLA are not particularly limited, so long as they bind to HLA. Mouse antibodies, rat antibodies, rabbit antibodies, sheep antibodies, human antibodies, and such may be used as necessary. Alternatively, artificially modified genetically recombinant antibodies, such as chimeric and humanized antibodies, may be used to reduce heterologous antigenicity against human. These modified antibodies can be produced using known methods. A chimeric antibody is an antibody comprising the heavy and light chain variable regions of an antibody from a non-human mammal such as mouse, and the heavy and light chain constant regions of a human antibody. The chimeric antibody can be produced by linking a DNA encoding mouse antibody variable regions with a DNA encoding human antibody constant regions, incorporating this into an expression vector, and then introducing the vector into a host.

The present inventors discovered that the antibodies of the present invention induce cell death. Based on this finding, the present invention provides cell death-inducing agents and cell growth inhibitors comprising an antibody of the present invention as an active ingredient. The present inventors previously discovered that diabodies prepared by reducing the molecular weight of an anti-HLA antibody have an anti-tumor effect against a human myeloma model animal (WO2004/033499). Furthermore, the cell death-inducing activity of the antibodies of the present invention is considered to have a significant effect, particularly in activated T cells or B cells. Accordingly, antibodies of the present invention would be particularly effective for treating or preventing tumors such as cancers (specifically hematopoietic tumors) and autoimmune diseases. The present invention also provides anti-tumor agents or therapeutic agents for autoimmune diseases, comprising an antibody of the present invention as an active ingredient.

Furthermore, the present invention provides cell death-inducing agents and cell growth-suppressing agents comprising antibodies of the present invention as an active ingredient. The cell death-inducing activity of the antibodies in the present invention is considered to have a particularly large effect on activated T cells or B cells; therefore, it is considered to be particularly effective for treatment and prevention of tumors such as cancer (particularly hematopoietic tumors) and autoimmune diseases. Accordingly, the present invention provides methods of treatment and prevention that use the antibodies of the present invention for tumors such as cancer (particularly hematopoietic tumors) and autoimmune diseases. When using antibodies whose molecular weight has not been reduced as active ingredients, they are preferably cross-linked with an anti-IgG antibody and such.

The pharmaceutical agents of the present invention can be used in combination with an interferon. Combined use of an anti-HLA class I antibody with interferon strongly enhanced anti-HLA class I antibody activities such as cell death induction and the like (WO2006/123724).

Generally, interferon is a generic term for a protein or glycoprotein that has antiviral action and is induced from animal cells by viruses, double stranded RNA, lectin, and such. In addition to antiviral action, interferons have cell growth-suppressing action and immunoregulatory action. They are categorized into several types according to the cells producing them, binding ability to specific receptors, and biological and physicochemical characteristics. The major types are α, β, and γ, and other types that are known to exist are IFNω, and IFNτ. Furthermore, 20 or more subtypes of interferon α are known to exist. At present, not only the naturally-derived formulations but also various genetically recombinant type formulations, such as PEG-interferon and consensus interferon and the like have been developed and are commercially available.

Interferon of the present invention may be any one of the above-mentioned types, but it is preferably α or γ. Furthermore, so long as the induction of cell death by anti-HLA class I antibody is enhanced, the interferon of the present invention may be any one of the naturally-derived type, artificially modified genetically-recombinant type, naturally-existing mutants, fusion proteins, or fragments thereof. Without particular limitation, the interferon of the present invention can be derived from, for example, humans, chimpanzees, orangutans, dogs, horses, sheep, goats, donkeys, pigs, cats, mice, guinea pigs, rats, rabbits, or such, or from other mammals. The interferon is preferably a human-derived interferon.

In the present invention, combined use of the antibodies of the present invention with an interferon means administering or using (hereinafter, simply referred to as "administering") the antibodies of the present invention together with an interferon, and there is no limitation on the order of administration or interval between administrations. The order in which an antibody of the present invention and interferon are administered may be administering an antibody of the present invention after administering interferon, administering an antibody of the present invention and interferon at the same time, or administering an antibody of the present invention before administering interferon, but is preferably, administering an antibody of the present invention after administering interferon, or administering an antibody of the present invention and interferon at the same time, and is more preferably administering an antibody of the present invention before administering interferon.

When administering an antibody of the present invention after administering interferon, the interval between administrations of the interferon and the antibody of the present invention is not particularly limited, and it can be set by taking factors such as route of administration and dosage form into consideration. An example of an administration interval is usually 0 hours to 72 hours, preferably 0 hours to 24 hours, and more preferably 0 hours to 12 hours.

An antibody of the present invention can be made into a single pharmaceutical composition with an interferon. Furthermore, antibodies of the present invention can be made into pharmaceutical compositions characterized by combined use with an interferon.

The pharmaceutical agents of the present invention can be administered in the form of a pharmaceutical, and can be administered orally or parenterally and systemically or topically. For example, intravenous injection such as drip infusion, intramuscular injection, intraperitoneal injection, subcutaneous injection, suppository, colonic infusion, or oral enteric coating agent may be selected, and a suitable administration method can be selected according to the age and symptoms of the patient. The effective dose can be selected from the range of 0.01 mg to 100 mg per kg body weight in each administration. Alternatively, the dosage can be selected from 1-1000 mg per patient, or preferably 5-50 mg per patient. For example, in the case of an HLA-recognizing antibody, preferred dose and method of administration refer to an effective dose, which is an amount that causes free antibodies to be present in the blood, and specific examples include administration methods such as administering 0.5 mg to 40 mg per month (four weeks) per kg body weight, which is preferably one mg to 20 mg in one to several doses, for example, by methods including intravenous injection such as drip infusion or subcutaneous injection following an administration schedule of twice/week, once/week, once/two weeks, once/four weeks, or such. The administration schedule can be adjusted by extending the administration interval from twice/week or once/week to once/two weeks, once/three weeks, or once/four weeks by observing post-administration condition and changes in blood test values.

Pharmaceutically acceptable carriers such as preservatives and stabilizers can be added to the pharmaceutical agents of the present invention. "Pharmaceutically acceptable carrier" refers to a carrier that itself may be a material that has or does not have the above-described activity, and the carrier is a pharmaceutically acceptable material that can be administered together with the above-mentioned pharmaceutical agent. Furthermore, it may be a material that does not have the above-mentioned activity, or a material that has a synergistic or additive effect when used in combination with an anti-HLA antibody.

Examples of pharmaceutically acceptable materials include sterilized water, physiological saline, stabilizers, excipients, buffers, preservatives, surfactants, chelating agents (for example, EDTA), and binders and the like.

In the present invention, about 0.2% gelatin or dextran, 0.1-1.0% sodium glutamate, approximately 5% lactose, or approximately 2% sorbitol, or such may be used as a stabilizer, without being limited thereto. Typical examples of preservatives include approximately 0.01% thimerosal, approximately 0.1% beta-propiolactone and such.

In the present invention, examples of surfactants include non-ionic surfactants. Typical examples include, sorbitan fatty acid esters such as sorbitan monocaprilate, sorbitan monolaurate, or sorbitan monopalmitate; glycerol fatty acid esters such as glycerol monocaprilate, glycerol monomyristate, or glycerol monostearate; polyglycerol esters of fatty acids such as decaglyceryl monostearate, decaglyceryl distearate, or decaglyceryl monolinoleate; polyoxyethylene sorbitan fatty acid esters such as polyoxyethylene sorbitan monolaurate, polyoxyethylene sorbitan monooleate, polyoxyethylene sorbitan monostearate, polyoxyethylene sorbitan monopalmitate, polyoxyethylene sorbitan trioleate, or polyoxyethylene sorbitan tristearate; polyoxyethylene sorbitol fatty acid esters such as polyoxyethylene sorbitol tetrastearate, polyoxyethylene sorbitol tetraoleate; polyoxyethylene glycerol fatty acid esters such as polyoxyethylene glyceryl monostearate; polyethylene glycol fatty acid esters such as polyethylene glycol distearate; polyoxyethylene alkyl ether such as polyoxyethylene lauyl ether; polyoxyethylene polyoxypropylene alkyl ether such as polyoxyethylene polyoxypropylene glycol, polyoxyethylene polyoxypropylene propylether, or polyoxyethylene polyoxypropylene cetyl ether; polyoxyethylene alkylphenyl ether such as polyoxyethylene nonylphenyl ether; polyoxyethylene hardened castor oils such as polyoxyethylene castor oil, or polyoxyethylene hardened castor oil (polyoxyethylene hydrogenated castor oil); polyoxyethylene beeswax derivatives such as polyoxyethylene sorbitol beeswax; polyoxyethylene lanolin derivatives such as polyoxyethylene lanolin; and polyoxyethylene fatty acid amide with HLB 6 to 18 such as polyoxyethylene stearylamide.

Anionic surfactants can also be listed as surfactants. Typical examples of anionic surfactants may include alkyl sulfate salts having an alkyl group of ten to 18 carbon atoms, such as sodium cetyl sulfate, sodium lauryl sulfate, or sodium oleyl sulfate; polyoxyethylene alkylether sulfate salts whose average mole of ethyleneoxide added is two to four and the number of carbon atoms in the alkyl group is 10 to 18, such as sodium polyoxyethylene lauryl sulfate; alkyl sulfosuccinate ester salts of eight to 18 carbon atoms in the alkyl group, such as sodium lauryl sulfosuccinate ester; naturally-occurring surfactants such as lecithin or glycerol lipid phosphate; sphingophospholipids such as sphingomyelin; and sucrose fatty acid esters of 12 to 18 carbon atoms in the fatty acid.

One or a combination of two or more of these surfactants can be added to the pharmaceutical agents of the present invention. Preferred surfactant to be used in the formulation of the present invention is a polyoxyethylene sorbitan fatty acid ester such as Polysorbate 20, 40, 60, 80, or such, and Polysorbate 20 and 80 are particularly preferred. Polyoxyethylene polyoxypropylene glycol represented by Poloxamer (for example, Pluronic F-68 ®) is also preferred.

The amount of surfactant added differs depending on the type of surfactant used, but for Polysorbate 20 or Polysorbate 80, it is generally 0.001-100 mg/mL, preferably 0.003-50 mg/mL, and more preferably 0.005-2 mg/mL.

Examples of buffers in the present invention include phosphoric acid, citric acid buffer, acetic acid, malic acid, tartaric acid, succinic acid, lactic acid, calcium phosphate, gluconic acid, caprylic acid, deoxycholic acid, salicylic acid, triethanolamine, fumaric acid, other organic acids, carbonic acid buffer, Tris buffer, histidine buffer, imidazole buffer and the like.

Solution formulations can be prepared by dissolving into aqueous buffers that are known in the field of solution formulation. The buffer concentration is generally 1-500 mM, preferably 5-100 mM, and even more preferably 10-20 mM.

Furthermore, the pharmaceutical agents of the present invention may include other low-molecular-weight polypeptides, proteins such as serum albumin, gelatin, and immunoglobulin, amino acids, sugars and carbohydrates such as polysaccharides and monosaccharides, and sugar alcohols.

Examples of amino acids in the present invention include basic amino acids such as arginine, lysine, histidine, and ornithine, and inorganic salts of these amino acids (preferably in the form of chloride salts or phosphate salts, or more specifically amino-acid phosphates). When using free amino acids, the pH is adjusted to a preferred value by adding a suitable physiologically acceptable buffer such as inorganic acids, particularly hydrochloric acid, phosphoric acid, sulfuric acid, acetic acid, formic acid, or a salt thereof. In such cases, the use of a phosphoric acid salt is particularly useful because a particularly stable freeze-dried product can be obtained. It is particularly advantageous when the preparation does not substantially contain an organic acid such as malic acid, tartaric acid, citric acid, succinic acid, or fumaric acid, or when a corresponding anion (malate ion, tartrate ion, citrate ion, succinate ion, fumarate ion, or such) is not present. Preferred amino acids are arginine, lysine, histidine, or ornithine. Furthermore, acidic amino acids such as glutamic acid and aspartic acid, and salts thereof (preferably sodium salts); neutral amino acids such as isoleucine, leucine, glycine, serine, threonine, valine, methionine, cysteine, or alanine; or aromatic amino acids such as phenylalanine, tyrosine, tryptophan, or derivative *N*-acetyltryptophan can be used.

Examples of sugars and carbohydrates such as polysaccharides and monosaccharides in the present invention include dextran, glucose, fructose, lactose, xylose, mannose, maltose, sucrose, trehalose, and raffinose.

Examples of sugar alcohols in the present invention include mannitol, sorbitol, inositol and such.

Aqueous solutions used for injections include, for example, physiological saline and isotonic solutions comprising glucose or other adjunctive agents such as D-sorbitol, D-mannose, D-mannitol, and sodium chloride. They may also be combined with appropriate solubilizing agents, such as alcohol (for example, ethanol), polyalcohol (for example, propylene glycol or PEG), or non-ionic surfactant (for example, polysorbate 80 or HCO-50).

If desired, diluents, solubilizing agents, pH-adjusting agents, soothing agents, sulfur-containing reducing agents, and antioxidants may be included.

Examples of sulfur-containing reducing agents in the present invention include *N-*acetyl cysteine, *N-*acetyl homocysteine, thioctic acid, thiodiglycol, thioethanolamine, thioglycerol, thiosorbitol, thioglycolic acid, and salts thereof, sodium thiosulfate, glutathione, and compounds carrying a sulfhydryl group such as thioalkanoic acid of one to seven carbon atoms.

Examples of antioxidants in the present invention include erythorbic acid, dibutylhydroxytoluene, butylhydroxyanisole, α-tocopherol, tocopherol acetate, L-ascorbic acid and salts thereof, L-ascorbyl palpitate, L-ascorbyl stearate, sodium bisulfite, sodium sulfite, triamyl gallate, propyl gallate, and chelating agents such as ethylenediamine tetraacetic acid disodium (EDTA), sodium pyrophosphate, and sodium metaphosphate.

If necessary, the pharmaceutical agents can be contained within microcapsules (microcapsules made of hydroxymethylcellulose, gelatin, poly[methyl methacrylate], or such), or made into colloidal drug delivery systems (such as liposomes, albumin microspheres, microemulsion, nanoparticles, and nanocapsules) (see for example, "Remington's Pharmaceutical Science 16th edition", Oslo Ed., 1980). Methods for preparing the pharmaceutical agents as controlled-release pharmaceutical agents are also well known, and such methods may be applied to the present invention (Langer et al., J. Biomed. Mater. Res. 1981, 15: 167-277; Langer, Chem. Tech. 1982, 12: 98-105; U.S. Patent No. 3,773,919; European Patent (EP) Patent Application No. 58,481; Sidman et al., Biopolymers 1983, 22: 547-556; EP 133,988).

Pharmaceutically acceptable carriers used are suitably selected from those mentioned above or combinations thereof according to the dosage form, but are not limited thereto.

When preparing injections, pH-adjusting agents, buffers, stabilizers, preservatives or such are added as necessary to prepare subcutaneous, intramuscular, and intravenous injections by common procedures. An injection can be prepared as a solid preparation for formulation immediately before use by freeze-drying a solution stored in a container. A single dose can be stored in a container or multiple doses may be stored in a same container.

A variety of known methods can be used as methods for administering the pharmaceutical agents of the present invention.

In the present invention, "to administer" includes administering orally or parenterally. Oral administration includes administration in the form of oral agents, and the dosage form for oral agents can be selected from granules, powders, tablets, capsules, dissolved agents, emulsion, suspension, and such.

Parenteral administration includes administration in the injectable form, and examples of an injection include intravenous injection such as drip infusion, subcutaneous injection, intramuscular injection, and intraperitoneal injection. Furthermore, effects of the methods of the present invention can be accomplished by introducing a gene comprising an oligonucleotide to be administered into a living organism using gene therapy techniques. The pharmaceutical agents of the present invention can be administered locally to a region to be treated. For example, the agents can be administered by local infusion or by the use of a catheter during surgery, or by targeted gene delivery of DNA encoding an inhibitor of the present invention.

Administration to patients may be performed, for example by intra-arterial injection, intravenous injection, or subcutaneous injection, alternatively by intranasal, transbronchial, intramuscular, transdermal, or oral administration using methods well known to those skilled in the art. Doses vary depending on the body weight and age of the patient, method of administration and such; nevertheless, those skilled in the art can appropriately select suitable doses. Furthermore, if a compound can be encoded by a DNA, the DNA may be incorporated into a gene therapy vector to carry out gene therapy. Doses and administration methods vary depending on the body weight, age, and symptoms of patients, but, again, they can be appropriately selected by those skilled in the art.

A single dose of the pharmaceutical agents of this invention varies depending on the target of administration, the target organ, symptoms, and administration method. However, an ordinary adult dose (with a body weight of 60 kg) in the form of an injection is approximately 0.1 to 1000 mg, preferably approximately 1.0 to 50 mg, and more preferably approximately 1.0 to 20 mg per day, for example.

When administered parenterally, a single dose varies depending on the target of administration, the target organ, symptoms, and administration method; however in the form of an injection, for example, a single dose of approximately 0.01 to 30 mg, preferably approximately 0.1 to 20 mg, and more preferably approximately 0.1 to 10 mg per day may be advantageously administered intravenously to an ordinary adult (with a body weight of 60 kg). For other animals, a converted amount based on the amount for a body weight of 60 kg, or a converted amount based on the amount for a body surface area can be administered.

Suitable inoculation method is determined by considering the type of pharmaceutical agent, the type of subject to be inoculated, and such. Containers that can be used are vials and pre-filled syringes. If necessary, solutions or powdered products produced by freeze-drying may be used. The product may be for single inoculation or multiple inoculations. The dose may vary depending on the method of administration, the type, body weight, and age of the subject to be inoculated, and such, but a suitable dose can be selected appropriately by those skilled in the art.

All patents, published patent applications, and publications cited herein are incorporated by reference in their entirety.

### Examples

Hereinbelow, the present invention will be specifically described with reference to Examples, but it is not to be construed as being limited thereto.

### [Example 1] Establishment of HLA class IA/β2M-expressing Ba/F3 cell lines

Ba/F3 cells that express human HLA class IA and human β2M were established.

First, a full-length HLA class IA (HLA-full)-expressing vector was prepared as follows. A gene fragment encoding full-length HLA class IA was amplified by performing PCR using a cDNA encoding the full-length HLA class IA as template and the following primers (sHLA-1 and fHLA-3'):
sHLA-1: TCC GAA TTC CAC CAT GGC CGT CAT GGC GCC CCG AAC (SEQ ID NO: 13); and
fHLA-3': TTG CGG CCG CTC ACA CTT TAC AAG CTG TGA GAG ACA (SEQ ID NO: 14).

The obtained DNA fragment was digested with EcoRI/NotI, and inserted into the EcoRI/NotI gap of the animal cell expression vector pCXND3 to construct a full-length HLA class IA (fHLA-A) expression vector (pCXND3-HLA-full).

Next, a full-length β2-microglobulin (β2M)-expressing vector was produced as follows. A full-length β2-encoding gene fragment was amplified by performing PCR using a cDNA derived from human spleen (human spleen cDNA, Clontech #S1206) as template and the following primers (β2M-1 and β2M-2):
p2M-1: AAG CGG CCG CCA CCA TGT CTC GCT CCG TGG C (SEQ ID NO: 15); and
β2M-2: TTT CTA GAT TAC ATG TCT CGA TCC CAC TTA ACT (SEQ ID NO: 16).

The obtained DNA fragment was digested with NotI/XbaI, and inserted into the NotI/XbaI site of pCOS2-ZEO to construct a full-length β2M expression vector (pCOS2zeo-β2M).

Next, HLA-A/β2M-expressing Ba/F3 cell lines were established as follows. Twenty µg each of pCXND3-HLA-full and pCOS2zeo-β2M were digested with PvuI, and then introduced into Ba/F3 cells suspended in PBS(-) (1 x 10⁷ cells/mL, 800 µL) by electroporation (BIO-RAD Gene Pulser, 0.33 kV, 950 µF, time const. 27.0). Cells were diluted to a suitable number in a growth medium (RPMI1640 + 10% FCS + P/S + 1 ng/mL IL-3) and plated onto a 96-well plate, and the following day, G418 and Zeocin were added to the cells at 400 µg/mL and 800 µg/mL, respectively. Thereafter, half of the medium was exchanged every three to four days, and ten days later, single clones were selected.

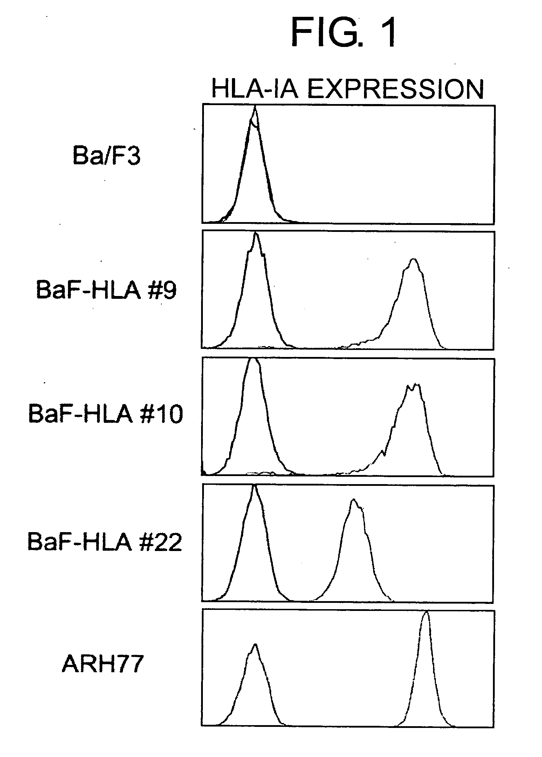

Expression levels of HLA class IA in the obtained HLA-A/β2M-expressing Ba/F3 cell lines (#9, #10, and #22) and ARH77 cells were determined by staining with 2D7 IgG (10 µg/mL), and expression of the antigens on the cell membrane was analyzed by FACS (COULTER, ELITE) (Fig. 1). As a result, the expression of HLA class IA in cell line #9 was found to be at the same level as in ARH77 cells. Therefore, this cell line was cultured in a large scale in RPMI1640 medium containing 1 ng/mL IL-3, 500 µg/mL G418, 800 µg/mL Zeocin (Invitrogen #46-0072), and 10%FCS, and this was used for cellular immunization.

### [Example 2] Cellular immunization

The HLA-A/β2M-expressing Ba/F3 cell line, BaF-HLA #9, was washed twice with PBS(-), suspended in PBS(-) to a concentration of 1.5-2.0 x 10⁷ cells/200 µL, and mice (MRL/lpr, male, four-weeks old, Japan Charles River) were immunized by intraperitoneally injecting 200 µL of this suspension (1-mL Terumo syringe, 26 G needle).

Immunization was carried out once a week for a total of eight immunizations, and the ninth immunization, which is the final immunization, was carried out using 200 µL of a 2.5 x 10⁷ cells/200 µL-suspension, and cell fusion was performed four days after the final immunization.

### [Example 3] Production of hybridomas

Spleens were aseptically removed from mice and homogenized in medium 1 [RPMI1640(+P/S)] to produce a single-cell suspension. This was passed through a 70-µm nylon mesh (Falcon) to remove adipose tissues and such, and the number of cells was counted. The obtained B cells were mixed with mouse myeloma cells (P3U1 cells) at a cell count ratio of about 2:1, 1 mL of 50% PEG (Roche, cat #: 783 641, lot #: 14982000) was added and cell fusion was performed. The fused cells were suspended in medium 2 [RPMI1640(+P/S, 10% FCS)] and dispensed at 100 µL/well into a suitable number (ten) of 96-well plates, and were incubated at 37°C. The following day, 100 µL/well of medium 3 [RPMI1640(+P/S, 10% FCS, HAT(Sigma, H0262), 5% BM Condimed H1 (Roche, cat #: 1088947, lot #: 14994800))] was added, and thereafter, 100 µL of the medium was removed from each well and 100 µL/well of fresh medium 3 was added every day for four days.

### [Example 4] Screening for cell death-inducing antibodies

Screening for hybridomas having cell death-inducing activity was carried out approximately one week after cell fusion. Screening for cell death-inducing antibodies was carried out as follows using the ability to induce cell aggregation as an indicator.

HLA-A/β2M-expressing Ba/F3 cells were plated onto a 96-well plate at 2.5 x 10⁴ cells/well, 80 µL of the culture supernatant of each hybridoma was added, and the cells were cultured at 37°C for 1 hour. Thereafter, anti-mouse IgG antibody (Cappel #55482, #55459) was added to a concentration of 6 µg/mL. After four more hours of incubation to carry out a cross-linking reaction, the cells were observed microscopically, and wells showing cell aggregation were selected. As a result of screening the culture supernatant of 1000 clones, ten positive hybridomas were obtained. Cells from these positive wells were plated again onto a 96-well plate at 2.5 cells/well and cultured for approximately ten days, and cell aggregation-inducing activity was analyzed again. This operation yielded ten types of single clones.

### [Example 5] Antibody panel production

### 5-1. Purification of antibodies

Antibodies were purified from 80 mL of the hybridoma culture supernatants of the obtained clones using a 1mL HiTrap Protein G HP column (Amersham Biosciences #17-0404-01). The hybridoma supernatants were adsorbed at a flow rate of 1 mL/min, and after washing with 20 mL of 20 mM phosphate buffer (pH7.0), elution was performed using 3.5 mL of 0.1 M Glycine-HCl (pH2.7). The eluted fractions were collected at 0.5 mL per tube in Eppendorf tubes preloaded with 50 µL of 1 M Tris-HCl (pH9.0). OD_{280 nm} was measured, antibody-containing fractions were combined, PBS(-) was added so that the total volume was 2.5 mL, and then the buffer was substituted to PBS(-) using a PD-10 column (Amersham Biosciences #17-0851-01). The purified antibodies were passed through a 0.22 µm filter (MILLIPORE #SLGV033RS), and the properties of each of the purified antibodies were examined in detail as described below.

### 5-2. Subtype determination

Determination of antibody subtypes was carried out using IsoStrip (Roche #1 493 027). For subtype determination, 10x PBS(-)-diluted hybridoma culture supernatants were used.

### 5-3. Epitope analysis

### 5-3-1. Cloning of the mouse MHC class IA gene

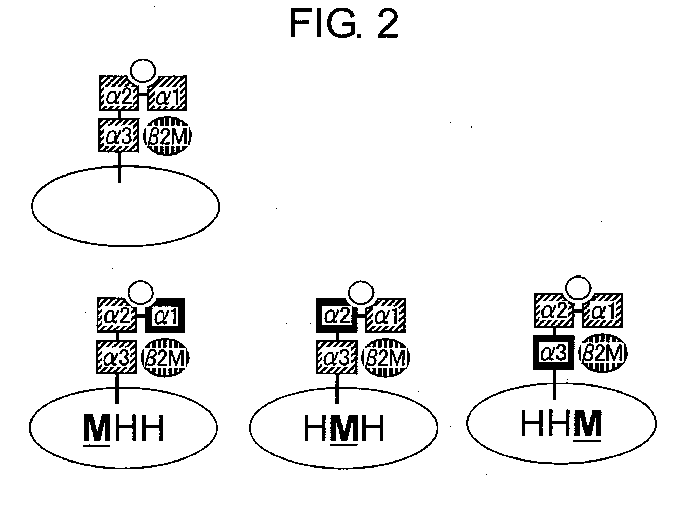

To analyze which domains of the HLA class IA molecule are recognized by the obtained antibodies, cell lines expressing chimeric HLA class IA that has one of the HLA class IA domains (α1 domain, α2 domain, and α3 domain) substituted with a corresponding mouse MHC class I domain were established as follows (Fig. 2).

First, cloning was carried out by the following method using the mouse MHC class IA gene as a template.

PCR was performed on mouse spleen cDNA (MTC panel, Clontech) using the following primers (mHLA-1 and mHLA-2) and pyrobest DNA polymerase (TAKARA #R005) to amplify the mouse HLA class IA gene fragment.
mHLA-1: CTG CTC CTG CTG TTG GCG GC (SEQ ID NO: 17)
mHLA-2: CAG GGT GAG GGG CTC AGG CAG (SEQ ID NO: 18)

The obtained gene fragment was TA-cloned into pCRII-TOPO (Invitrogen TOPO TA-cloning kit, #45-0640) to confirm its nucleotide sequence.

### 5-3-2. Construction of chimeric HLA-A expression vectors for use in epitope analysis

Next, chimeric HLA-A expression vectors to be used for epitope analysis were constructed as follows.

The MHH expression vector, pCOS2-chHLA-MHH flag, in which the HLA-A α1 domain is from a mouse (MHH), was constructed by the following method.

The HLA-A signal sequence (fragment A) was amplified by PCR using pyrobest DNA polymerase (TAKARA #R005) and an expression vector carrying the full-length HLA-A (pCXND3-HLA full) as template, with the following primers (sHLA-A and chHLA-H1):
sHLA-A: TCC GAA TTC CAC CAT GGC CGT CAT GGC GCC CCG AAC (including EcoRI site) (SEQ ID NO: 19); and
chHLA-H1: AAT CTA GAC TGG GTC AGG GCC AGG GCC CC (including XbaI site) (SEQ ID NO: 20).

The sequence from the α2 domain to the stop codon of HLA-A (fragment B) was amplified by PCR using the following primers (chHLA-H2 and chHLA-H3):
chHLA-H2: TTT CTA GAG CCG GTT CTC ACA CCA TCC AGA GG (including XbaI site) (SEQ ID NO: 21); and
chHLA-H3: AAG GAT CCC ACT TTA CAA GCT GTG AGA GAC ACA T (including BamHI site) (SEQ ID NO: 22).

Fragment A and fragment B were digested with Eco-RI-XbaI and XbaI-BamHI, respectively, and these fragments were inserted into the EcoRI-BamHI site of pCOS2-FLAG The nucleotide sequence of the obtained plasmid was confirmed and pCOS2-(M)HH was constructed.

At the same time, the α1 domain of mouse MHC class IA (fragment C) was amplified by PCR using pyrobest DNA polymerase (TAKARA #R005) and the mouse MHC class IA gene as template, with the following primers (chHLA-M1 and chHLA-M2):
chHLA-M1: TTT CTA GAG CGG GCC CAC ATT CGC TGA GG (including XbaI site) (SEQ ID NO: 23); and
chHLA-M2: TTT CTA GAC TGG TTG TAG TAT CTC TGT GCG GTC C (including XbaI site) (SEQ ID NO: 24).

The obtained fragment C was digested with XbaI, and this was inserted into pCOS2-(M)HH opened with XbaI. The nucleotide sequence was confirmed, and the construction of pCOS2-chHLA-MHH-flag, an expression vector in which the α1 domain is substituted with mouse MHC-A was completed.

The HMH expression vector, pCOS2-chHLA-HMH flag, in which the α2 domain is from a mouse (HMH), was constructed by the following method.

The HLA-A signal sequence-α1 domain (fragment D) was amplified by PCR using pyrobest DNA polymerase (TAKARA #R005) and an expression vector carrying the full-length HLA-A (pCXND3-HLA full) as template, with the following primers (sHLA-A and chHLA-H4):
sHLA-A: TCC GAA TTC CAC CAT GGC CGT CAT GGC GCC CCG AAC (including EcoRI site) (SEQ ID NO: 19); and
chHLA-H4: TTG TCG ACC CGG CCT CGC TCT GGT TGT AGT AG (including SalI site) (SEQ ID NO: 25).

The following primers (chHLA-H5 and chHLA-H3) were used to amplify from α3 domain to the stop codon of HLA-A (fragment E) by PCR.
chHLA-H5: AAG TCG ACG CCC CCAAAA CGC ATA TGA CT (including SalI site) (SEQ ID NO: 26); and
chHLA-H3: AAG GAT CCC ACT TTA CAA GCT GTG AGA GAC ACA T (including BamHI site) (SEQ ID NO: 22).

Fragment D and fragment E were digested with EcoRI-SalI and SalI-BamHI, respectively, and these fragments were inserted into the EcoRI-BamHI site of pCOS2-FLACz The nucleotide sequence of the obtained plasmid was confirmed and pCOS2-H(M)H was constructed.

At the same time, the α2 domain of mouse MHC class IA (fragment F) was amplified by PCR using pyrobest DNA polymerase (TAKARA #R005) and the mouse MHC class IA gene as template, with the following primers (chHLA-M3 and chHLA-M4):
chHLA-M3: TTG TCG ACC ACG TTC CAG CGG ATG TTC GGC (including SalI site) (SEQ ID NO: 27); and
chHLA-M4: GAG TCG ACG CGC AGC AGC GTC TCA TTC CCG (including SalI site) (SEQ ID NO: 28).

The obtained fragment F was digested with SalI, and this was inserted into pCOS2-H(M)H opened with SalI. The nucleotide sequence was confirmed, and the construction of pCOS2-chHLA-HMH-flag, an expression vector in which the α2 domain is substituted with mouse MHC-A was completed.

The HHM expression vector, pCOS2-chHLA-HHM flag, in which the α3 domain is from a mouse (HHM), was constructed by the following method.

The HLA-A signal sequence-α2 domain (fragment G) was amplified by PCR using pyrobest DNA polymerase (TAKARA#R005) and an expression vector carrying the full-length HLA-A (pCXND3-HLA full) as template, with the following primers (sHLA-A and chHLA-H6):
sHLA-A: TCC GAA TTC CAC CAT GGC CGT CAT GGC GCC CCG AAC (including EcoRI site) (SEQ ID NO: 19); and
chHLA-H6: TTT CTA GAG TCC GTG CGC TGC AGC GTC TCC T (including XbaI site) (SEQ ID NO: 29).

The intracellular domain of HLA-A (fragment H) was amplified by PCR using the following primers (chHLA-H7 and chHLA-H3):
chHLA-H7: TTT CTA GAA TGG GAG CCG TCT TCC CAG CCC A (including XbaI site) (SEQ ID NO: 30); and
chHLA-H3: AAG GAT CCC ACT TTA CAA GCT GTG AGA GAC ACA T (including BamHI site) (SEQ ID NO: 22).

Fragment G and fragment H were digested with EcoRI-XbaI and XbaI-BamHI, respectively, and these fragments were inserted into the EcoRI-BamHI site of pCOS2-FLAG The nucleotide sequence of the obtained plasmid was confirmed and pCOS2-HH(M) was constructed.

At the same time, the mouse MHC class IA α3 domain (fragment I) was amplified by PCR using pyrobest DNA polymerase (TAKARA #R005) and the mouse MHC class IA gene as template, with the following primers (chHLA-M5 and chHLA-M6):
chHLA-M5: AAT CTA GAA AGG CCC ATG TGA CCT ATC ACC CC (including XbaI site) (SEQ ID NO: 31); and
chHLA-M6: TAT CTA GAG TGA GGG GCT CAG GCA GCC CC (including XbaI site) (SEQ ID NO: 32).

The obtained fragment I was digested with XbaI, and this was inserted into pCOS2-HH(M) opened with XbaI. The nucleotide sequence was confirmed, and the construction of pCOS2-chHLA-HHM-flag, an expression vector in which the α3 domain is substituted with mouse MHC-A was completed.

The MMM expression vector, pCOS2-chHLA-MMM flag, in which the α1-α3 domains are from a mouse (MMM), was constructed by the following method.

Fragment A and fragment H were digested with EcoRI-XbaI and XbaI-BamHI, respectively, and these fragments were inserted into the EcoRI-BamHI site of pCOS2-FLAG. The nucleotide sequence of the obtained plasmid was confirmed and pCOS2-(MMM) was constructed.

The mouse MHC class IA α1-α3 domains (fragment J) was amplified by PCR using pyrobest DNA polymerase (TAKARA #R005) and the mouse MHC class IA gene as template, with the following primers (chHLA-M1 and chHLA-M6):
chHLA-M1: TTT CTA GAG CGG GCC CAC ATT CGC TGA GG (including XbaI site) (SEQ ID NO: 23); and
chHLA-M6: TAT CTA GAG TGA GGG GCT CAG GCA GCC CC (including XbaI site) (SEQ ID NO: 32).

The obtained fragment J was digested with XbaI, and this was inserted into pCOS2-(MMM) opened with XbaI. The nucleotide sequence was confirmed, and the construction of pCOS2-chHLA-MMM-flag, an expression vector in which the α1-α3 domains are substituted with mouse MHC-A was completed.

### 5-3-3. Establishment of chimeric HLA-A/β2M-expressing Ba/F3 cell lines for epitope analysis

Twenty µg each of pCOS2-chHLA-MHH-flag, pCOS2-chHLA-HMH-flag, pCOS2-chHLA-HHM-flag, and pCOS2-chHLA-MMM-flag were digested with PvuI, and then introduced into Ba/F3 cells suspended in PBS(-)(1 x 10⁷ cells/mL, 800 µL) by electroporation (BIO-RAD Gene Pulser, 0.33 kV, 950 µF, time const. 27.0). The cells were diluted to a suitable number in a growth medium (RPMI1640 + 10% FCS + P/S + 1 ng/mL IL-3) and plated onto a 96-well plate, and the following day, G418 was added to a concentration of 500 µg/mL. Ten days later, single clones were selected by microscopic observation.

With regard to these clones, 1 x 10⁵ cells were dissolved in 50 µL of 0.5% NP40 lysis buffer (10 mM Tris-HCl (pH7.5) containing 0.5% NP40, 150 mM NaCl, and 5 mM EDTA), and SDS-PAGE was carried out using 12 µL of the supernatant. After blotting onto a PVDF membrane, Western blotting was performed using Anti-Flag M2 antibody (SIGMA #F3165) and HRP-Anti-Mouse Antibody (Amersham Biosciences #NA9310) to screen for chHLA-producing cell lines. Those with the highest chHLA expression, chHLA-MHH #8, chHLA-HMH #6, chHLA-HHM #2, and chHLA-MMM #4, were selected, and placed in RPMI1640 medium containing 1 ng/mL IL-3, 500 µg/mL G418, and 10% FCS for large-scale culturing. pCOS2zeo-β2M digested with PvuI (15 µg) was introduced into each of these chHLA-expressing cell lines by electroporation. The following day, G418 and Zeocin (Invitrogen #46-0072) were added to a concentration of 500 µg/mL and 800 µg/mL, respectively. Twelve days later, single clones were selected by microscopic observation. These cells were stained using an anti-human β2M antibody (SIGMA #M7398) and anti-mouse IgG-FITC antibody (Beckman Coulter #IM0819), and expression of β2M on cell membrane was analyzed by FACS (Beckman Coulter, ELITE). Those with the highest β2M expression, chHLA-MHH/β2M #1-3, chHLA-HMH/β2M #2-1, chHLA-HHW/β2M #3-4, and chHLA-MMM/β2M #4-6, were placed in RPMI1640 medium containing 1 ng/mL IL-3, 500 µg/mL G418, 800 µg/mL Zeocin, and 10%FCS for large-scale culturing, and used for epitope analysis.

### 5-3-4. Epitope analysis by FACS

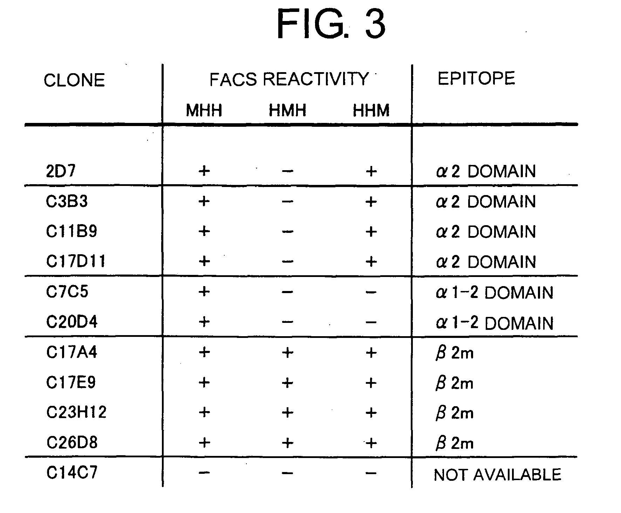

To determine the epitopes of the obtained antibodies (ten clones), their ability to bind to the chimeric HLA/β2M-expressing cells was analyzed. Chimeric HLA/β2M-expressing cells were plated onto a 96-well plate at 8 x 10⁵ cells/well and each of the antibodies was added to a concentration of 10 µg/mL. After one-hour incubation on ice, the cells were washed with 150 µL of FACS buffer, stained with an anti-mouse IgG-FITC antibody (Beckman Coulter #IM0819), and then analyzed by FACS (Beckman Coulter, ELITE) (Fig. 3).

As a result, since C3B3, C11B9, and C17D11 did not bind to HMH/Ba/F3 (which have a mouse HLA α2 domain), the epitope was found to be an α2 domain. On the other hand, although C17A4, C17E9, C23H12, and C26D8 did not cross over with mouse MHC class I (results not shown), they bound to all chimeric HLAs, and their FACS staining patterns matched the staining pattern observed with the anti-α2M antibody; therefore, it was determined that these clones react with β2M but not with HLA. Since C7C5 and C20D4 did not bind to the HLA of HMH (which have a mouse HLA α2 domain) or HHM (which have a mouse HLA α3 domain), these clones were inferred to recognize the region between α2 and α3.

### 5-4. Cell death-inducing activity

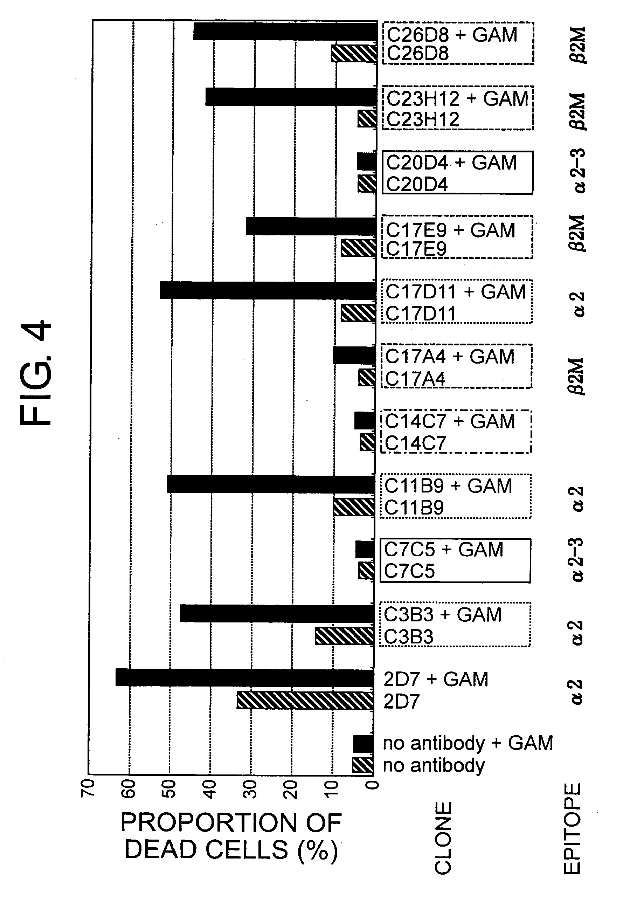

Cell death-inducing activity of the obtained antibodies on ARH77 cells was evaluated as follows. Each of the purified antibody (5 µg/mL) was added to ARH77 cells, and then the cells were cultured in the presence (120 µg/mL) or absence of secondary antibodies (anti-mouse IgG antibodies, Cappel #55482, #55459) at 37°C for four hours. After culturing, the cells were collected, stained with propidium iodide (PI), and the percentage of PI-positive cells (dead cells) was measured by FACS (Beckman Coulter, ELITE) (Fig. 4).

As a result, relatively strong cell death-inducing activity was confirmed for the C3B3, C17D11, and C11B9 antibodies in the presence of cross-linking.

### 5-5. Cloning of the variable region

Total RNA was purified from approximately 5x10⁶ hybridomas using RNeasy Mini Kit (QIAGEN #74104) and QIAshredder (QIAGEN #79654). From 1 µg of the total RNA, cDNAs were synthesized using SMART RACE cDNA Amplification Kit (CLONTECH #PT3269-1). The 5'-CDS primer included in the kit was used. Using the obtained cDNA as template, the heavy chain variable regions (V_{H}) and light chain variable regions (V_{L}) were amplified by PCR under the following conditions.
Primer: UPM ←→ G2a (V_{H}; IgG2a), UPM ←→ k(V_{L}; k)
94°C for 5 sec, 72°C for 2 min, 5 cycles
94°C for 5 sec, 70°C for 10 sec, 72°C for 2 min, 5 cycles
94°C for 5 sec, 68°C for 10 sec, 72°C for 2 min, 27 cycles

The obtained gene fragments were TA-cloned into pCRII-TOPO (Invitrogen TOPO TA-cloning kit, #45-0640), and the nucleotide sequences were confirmed. Sequences were confirmed by analyzing at least two or more plasmids per gene. The nucleotide sequence of the heavy chain variable region including the leader sequence confirmed in the present Example is shown in SEQ ID NO: 46, and the amino acid sequence of the heavy chain variable region encoding this nucleotide sequence is shown in SEQ ID NO: 47. The nucleotide sequence from nucleotides 58 to 432 of SEQ ID NO: 46 (SEQ ID NO: 1), and the amino acid sequence from amino acids 20 to 144 of SEQ ID NO: 47 (SEQ ID NO: 2) correspond to the heavy chain variable region.

Furthermore, the nucleotide sequence of the light chain variable region including the leader sequence confirmed in the present Example is shown in SEQ ID NO: 48, and the amino acid sequence of the light chain variable region encoding this nucleotide sequence is shown in SEQ ID NO: 49. The nucleotide sequence from nucleotides 61 to 381 of SEQ ID NO: 48 (SEQ ID NO:3), and the amino acid sequence from amino acids 21 to 127 of SEQ ID NO: 49 (SEQ ID NO: 4) correspond to the light chain variable region.

### 5-6. Production of an antibody panel

Information related to the obtained antibodies described above was summarized in a panel. The information includes isotype classification of the antibodies, genetic sequences encoding the variable regions of the antibodies, epitopes, binding activities against ARH77 cells, cell death-inducing activities, and such (Table 1).

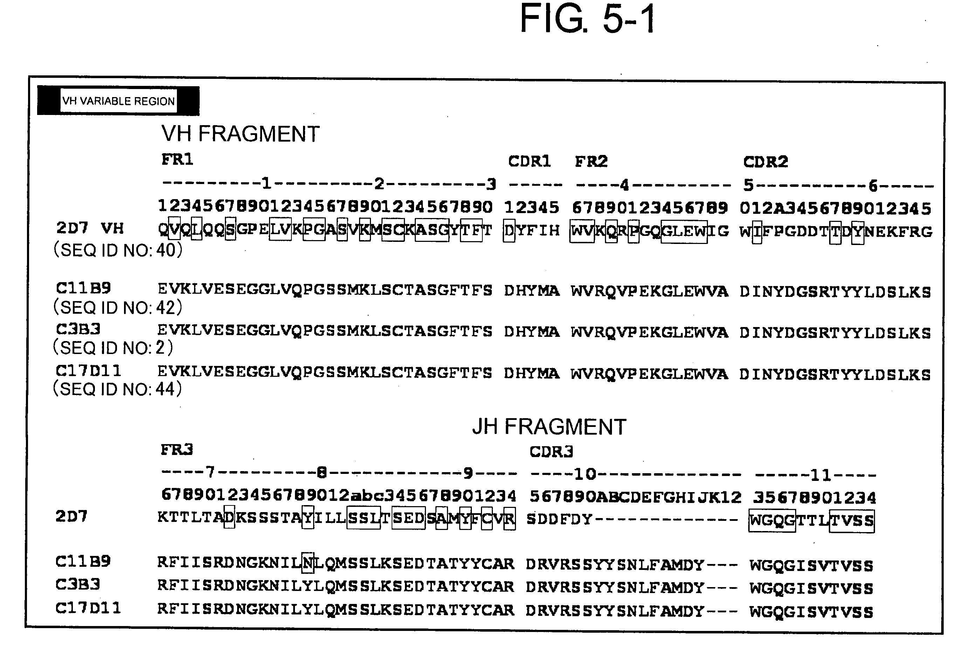

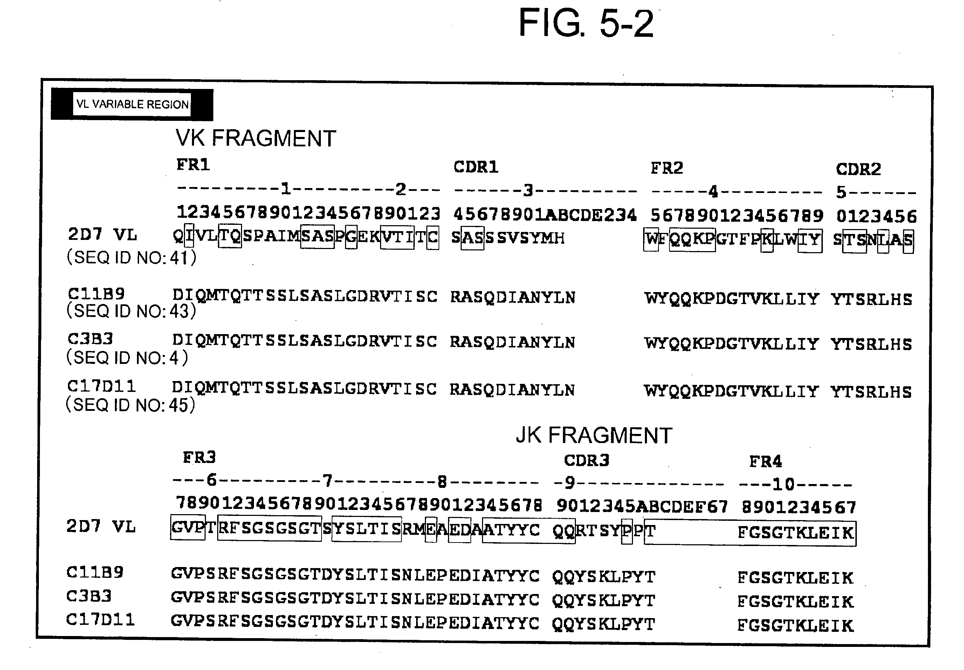

Results of analyzing the variable region-encoding amino acid sequences showed that among the heavy chain variable regions of the three clones (C3B3, C17D11, and C11B9) having the α2 domain as an epitope, C3B3 and C17D11 had the same amino acid sequence, but C 11B9 had a sequence differed by one amino acid from those of C3B3/C17D11 (Fig. 5-1). The sequences of the light chain variable regions were identical for all three clones (Fig. 5-2).

**[Table 1]**

| Group | Clone ID | Mouse Strain | Isotype | Epitope | Binding to ARH77 (X mode) | Cell death induction (proportion of dead cells (%)) | |
|---|---|---|---|---|---|---|---|
| | | | | | | Cross-link (-) | Cross-link (+) |
| | 2D7 | Balb/c | IgG2b | α2 | 98.8 | 33.4 | 63.6 |
| A | C3B3 | MRL/lpr | IgG2a | α2 | 74 | 14.3 | 47.7 |
| | C17D11 | MRL/lpr | IgG2a | α2 | 82 | 8.5 | 53.1 |
| | C11B9 | MRL/lpr | IgG2a | α2 | 71 | 10.2 | 51.1 |
| B | C23H12 | MRL/lpr | IgG2a | β2M | 69.6 | 4.8 | 42.3 |
| | C26D8 | MRL/lpr | IgG2a | β2M | 66.5 | 11.2 | 45.1 |
| | C17E9 | MRL/lpr | IgG2a | β2M | 69.6 | 8.7 | 32 |
| | C17A4 | MRL/lpr | IgG2a | β2M | 11.6 | 4.2 | 10.4 |
| C | C20D4 | MRL/lpr | IgG2a | α2/3 | 14.2 | 4.4 | 4.7 |
| | C7C5 | MRL/lpr | IgG1/2a | α2/3 | 7.8 | 4 | 4.8 |
| D | C14C7 | MRL/lpr | IgG1 | ? | 2.9 | 3.8 | 5.2 |

### [Example 6] Production of diabodies

### 6-1. Production of diabody vectors

Although the obtained C3B3 antibody showed cell death-inducing activity against ARH77 cells in the presence of a secondary antibody (GAM), the antibody alone did not show strong cell death-inducing activity. Therefore, a diabody in which the C3B3 antibody variable regions are linked by a 5-mer peptide (GGGGS) (SEQ ID NO: 33) was produced.

The portion from the signal sequence to FR4 of the H-chain variable region was amplified by PCR using V_{H} TA-cloned into pCRII-TOPO as a template and pyrobest DNA polymerase (TAKARA #R005). A 5' primer to which an EcoRI site is added and a 3' primer to which a linker sequence (amino acids GGGGS) is added were used.

Similarly, the sequence from FR1 to FR4 of the L chain variable region was amplified by PCR using V_{L} TA-cloned into pCRII-TOPO as template. A 5' primer to which a linker sequence (amino acid: GGGGS) is added and a 3' primer to which Flag tag and Not I site are added were used.

The amplified V_{H} and V_{L} were annealed to each other, and then the diabody gene was amplified by PCR using primers for both ends. The obtained fragment was digested with EcoRI/NotI and inserted into the EcoRI/NotI site of pCXND3. The nucleotide sequence was confirmed, and construction of the expression vector was completed. The primers and PCR reaction conditions used when producing the C3B3 diabody (linker: 5 amino acid) are shown below.
Primers:
C3B3DB-H1: cct gaa ttc CAC CAT GTA CTT CAG GCT CAG CTC AG (SEQ ID NO: 34)
C3B3DB-H2: GGA TAT Cgc tac cgc ctc cac cTG AGG AGA CGG TGA CTG AAA TTC CTT (SEQ ID NO: 35)
C3B3DB-L1: CAg gtg gag gcg gta gcG ATA TCC AGA TGA CAC AGA CTA CAT CCT CC (SEQ ID NO: 36)
C3B3DB-L2: att gcg gcc gct tat cac tta tcg tcg tca tcc ttg tag tcT TTT ATT TCC AGC TTG GTC CCC GAT CCG (SEQ ID NO: 37)
PCR reaction conditions:
94°C for 1 minute
94°C for 30 minutes, 72°C for 30 minutes, 25 cycles
Next, the obtained PCR products were purified using an S-300 HR column (Amersham Biosciences #27-5130-01), and 1 µL of V_{H} and V_{L} each were annealed under the following conditions using pyrobest DNA polymerase.
94°C for 1 minute
94°C for 30 minutes, 72°C for 30 minutes, 5 cycles
1 µL of the reaction solution obtained after annealing was subjected to PCR under the conditions below, using C3B3DB-5' and C3B3DB-3'which are shorter primers than C3B3DB-H1 and C3B3DB-L2.
C3B3DB-5': cct gaa ttc CAC CAT GTA CTT CAG GC (SEQ ID NO: 38)
C3B3DB-3': att gcg gcc gct tat cac tta tcg (SEQ ID NO: 39)
94°C for 1 minute
94°C for 30 minutes, 72°C for 1 minute, 25 cycles

The amplified fragments were purified using an S-400 HR column (Amersham Biosciences #27-5140-01), digested with EcoRI/NotI, and inserted into the EcoRI/NotI site of pCXND3. The inserted nucleotide sequence was confirmed, and construction of pCXND3-C3B3DB-Flag was completed. The nucleotide sequence of the diabody comprising a leader sequence and Flag-tag sequence, which was confirmed in the present Example, is shown in SEQ ID NO: 50, and the amino acid sequence of the diabody encoded by this nucleotide sequence is shown in SEQ ID NO: 51. In SEQ ID NO: 50, the nucleotide sequence from nucleotides 58 to 432 corresponds to the heavy chain variable region, the nucleotide sequence from nucleotides 433 to 447 correspond to the linker sequence, and the nucleotide sequence from nucleotides 448 to 768 corresponds to the light chain variable region. In SEQ ID NO: 51, the amino acid sequence of amino acids 20 to 144 corresponds to the heavy chain variable region, the amino acid sequence from amino acids 145 to 149 corresponds to the linker sequence, and the amino acid sequence from amino acids 150 to 256 corresponds to the light chain variable region.

### 6-2. Establishment of diabody-expressing cell lines

These vectors were introduced into DG44 cells to establish C3B3 diabody-producing cell lines. Ten µg of each of the diabody expression vectors was digested with PvuI and then introduced into DG44 cells suspended in PBS(-) (1 x 10⁷ cells/mL, 800 µL) by electroporation (BIO-RAD Gene Pulser, 1.5 kV, 25 µF). The cells were diluted to a suitable number in a growth medium (CHO-S-SFMII/PS), and plated onto a 96-well plate. The next day, G418 was added at a final concentration of 500 µg/mL. Approximately two weeks later, wells with a single clone were selected by observation under the microscope, and SDS-PAGE was carried out using 10 µL each of the culture supernatants. After blotting onto a PVDF membrane, Western blotting was performed using anti-Flag M2 antibody (SIGMA#F3165) and HRP-anti-mouse antibody (Amersham Biosciences # NA9310), and screening for diabody-producing cell lines was carried out. Scale-up culture was carried out for the cell line showing the highest production level.

### 6-3. Diabody purification

100 mL of the culture supernatant of the C3B3 diabody-expressing DG44 cell line was passed through a 0.22 µm filter (MILLIPORE #SLGV033RS), and then this was adsorbed onto a K9 column (Amersham Biosciences #19-0870-01) filled with 1 mL of ANTI-FLAG M2 Agarose Affinity Gel (SIGMA #A-2220) using a P 1 pump at a flow rate of 1 mL/min. After washing the column with 6 mL of 50 mM Tris-HCl (pH7.4), 150 mM NaCl, 0.01% Tween20, elution was carried out with 7 mL of 0.1 M Glycine-HCl (pH3.5), 0.01% Tween20. Washing and elution were carried out using AKTAexplorer 10S at a flow rate of 1 mL/min. While the absorbance at 280 nm was monitored, 0.5 mL eluted fractions were collected at a time into 5-mL tubes preloaded with 50 µL of 1 M Tris-HCl (pH 8.0). The collected fractions were combined, concentrated to 300 µL using Centricon YM-10 (amicon #4205), and then immediately subjected to gel filtration chromatography.

Gel filtration chromatography was performed using a Superdex 200 HR column (Amersham #17-1088-01) and AKTAexplorer 10S at a flow rate of 0.4 mL/min. After equilibration with 0.01% Tween20 in PBS(-), the above-mentioned M2-purified sample was injected manually. While the absorbance at 280 nm was monitored, 0.5 mL fractions were collected into 5 mL tubes. Fractions corresponding to each peak were combined, passed through a 0.22 µm filter (MILLIPORE #SLGV033RS or SLGV004SL), and then stored at 4°C.

### 6-4. Analysis of cell death-inducing activity of diabodies

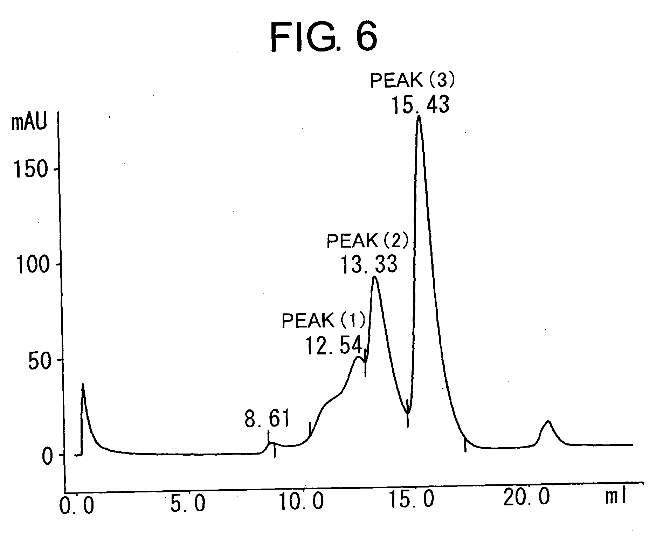

As indicated in the chart of Fig. 6, C3B3 minibodies were separated by gel filtration chromatography into three main fractions.(Peak (1), Peak (2), and Peak (3)) according to differences in molecular weight (three-dimensional structure). For each of these fractions, cell death-inducing activity was measured and compared with the cell death-inducing activity of 2D7 diabody.

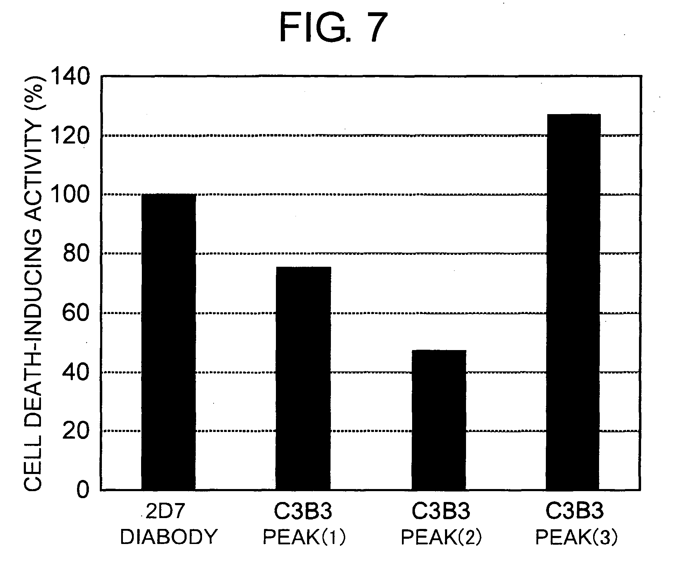

As a result, only weak cell death-inducing activities were observed in the high-molecular-weight fractions (Peaks (1) and (2): C3B3 multimers), but in the dimmer fraction (Peak (3): C3B3 diabody), strong cell death-inducing activity exceeding that of 2D7 daibody was observed (Fig. 7).

### 6-5. Comparison of growth suppressing effects between the purified C3B3 diabody and 2D7 diabody

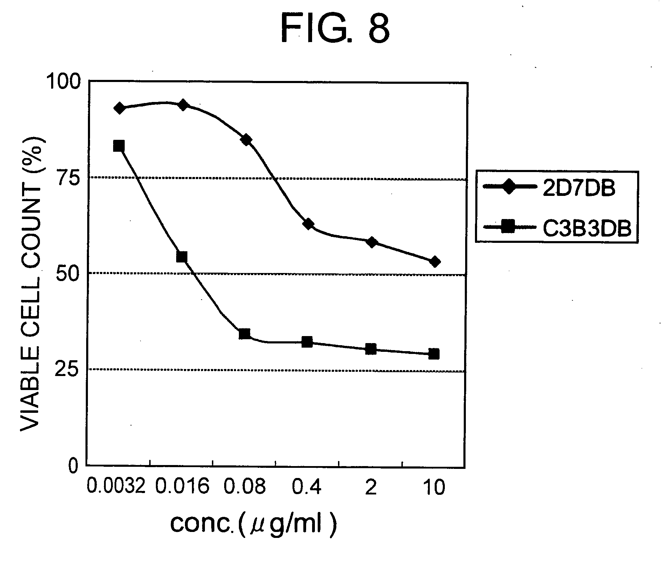

The growth suppressing abilities of the purified C3B3 diabody (Fig. 6, Peak (3)) and the currently known 2D7 diabody were compared. ARH77 cells were plated onto a 96-well plate at a cell concentration of 1-2 x 10⁴ cells/well, each of the obtained antibodies were added at a suitable concentration, and cell count measurements were taken after culturing for three days. Viable cell count was determined using WST-8 (viable cell count reagent SF; Nacalai Tesque). More specifically, after this reagent was added to the cells at 10 µL/well, the cells were cultured at 37°C for 1.5 hours, and the absorbance at 450 nm was measured on a spectrophotometer. The values were presented as relative viable cell count (Fig. 8).

As a result, C3B3 diabody showed strong growth-suppressing ability at a lower concentration compared with the 2D7 diabody. This proved that C3B3 diabody is a low-molecular-weight antibody having a stronger antitumor effect than the 2D7 diabody.

### [Example 7] Large-scale preparation of the C3B3 diabody

### 7-1. Preparation of culture supernatant

1 x 10⁷ C3B3 diabody-Flag-expressing DG44 cells were suspended in 2 L of CHO-S-SFMII (Invitrogen, c/n: 12052-098)/PS (Invitrogen, c/n: 15140-122) medium and were seeded in cellSTACK (Corning, c/n: 3271). Cells were cultured at 37°C in a 5% CO₂ incubator, and when the survival rate became less than 60% (cultured for approximately 7 days), the culture supernatant was collected. The collected culture supernatant was centrifuged at 3000 rpm for 20 minutes at 4°C, and the supernatant was passed through a 0.22 µm filter (Corning, c/n: 430513) and then stored at 4°C.

### 7-2. Purification by chromatography (1)

### 7-2-1. Coarse purification with an anion column

XK50 column was filled with Q Sepharose Fast Flow (Amersham Biosciences, c/n: 17-0510-01) (bed volume of 100 mL). This was washed sequentially with 500 mL of milliQ water and 500 mL of 20 mM Tris-HCl (pH7.5) containing 1 M NaCl and 0.01% Tween20 (QB), and then equilibrated with 500 mL of 20 mM Tris-HCl (pH7.5) containing 0.01 % Tween20 (QA). For a two-fold dilution, 2L of milliQ water was added to 2 L of culture supernatant, and after the pH was adjusted to 7.8 by adding approximately 20 mL of 1 M Tris, this was adsorbed onto the equilibrated column. Adsorption was carried out using a P1 pump, at a maximum flow rate of 10 mL/min at 4°C for approximately 15 hours. This was followed by washing and elution using AKTAprime at a flow rate of 10 mL/min. After washing the column with 300 mL of 16% QB, elution was carried out using 400 mL of 25% QB and 100 mL of 30% QB. Fractions of 12 mL were collected in 15-mL tubes. While the absorbance at 280 nm was monitored, fractions were collected from the first peak after switching to 25% QB to until 100 mL of 30% QB was passed.

After the collected fractions were combined and passed through a 0.22 µm filter (Corning, c/n: 430626), 0.6 equivalent of QA was added, the salt concentration was adjusted to approximately 150 mM, and this was stored at 4°C.

The column was washed sequentially with 400 mL of QB, 200 mL of 0.1 M NaOH, and 200 mL of QB, then equilibrated with 500 mL of QA for regeneration.

### 7-2-2. Purification by ANTI-FLAG M2 Affinity column (M2 column)

XK26 column was filled with ANTI-FLAG M2 Affinity Gel Freezer-Safe (SIGMA, c/n: A2220) (bed volume of 10 mL). This was washed with 50 mL of 50 mM Tris-HCl (pH7.4) containing 150 mM NaCl and 0.01% Tween20 (MA), and 30 mL of 0.1 M Glycine-HCl (pH3.5) containing 0.01 % Tween20 (MB), and then equilibrated with 50 mL of MA.

Next, 540 mL of the anion column-coarsely purified sample (corresponding to approximately 2 L of culture supernatant) was adsorbed onto two tandemly connected M2 columns. Adsorption was carried out using a P1 pump, at a maximum flow rate of 1 mL/min at 4°C for approximately 15 hours. This was followed by washing and elution using AKTAexplorer 10S at a flow rate of 4 mL/min. After washing the column with 50 mL of MA, elution was carried out using 30 mL of 100% MB. While the absorption at 280 nm was monitored, the eluate was collected in 2 mL each into 5-mL tubes preloaded with 200 µL of 1 M Tris-HCl (pH8.0). After combining the collected fractions and concentrating this to 5 mL using Centriprep YM-10 (amicon, c/n: 4304), this was immediately subjected to gel filtration for buffer exchange. When insoluble matter was found by visual observation, the solution was passed through a 0.22 µm filter (MILLIPORE, c/n: SLGV013SL) and then subjected to gel filtration.

After the sample was eluted, the column was equilibrated with 50 mL of MA, and then stored at 4°C. When the column was not going to be used for more than a week, 30 mL or more of 50 mM Tris-HCl (pH7.4) containing 150 mM NaCl and 0.02% NaN₃ was passed through the column, and this was stored at 4°C.

### 7-2-3. Purification by gel filtration chromatography

Gel filtration using HiLoad 26/60 Superdex 200 pg (Amersham, c/n: 17-1071-01) was performed to separate the diabody and carry out buffer exchange. This operation was performed using AKTAexplorer 10S at a flow rate of 2 mL/min. After equilibration with PBS(-) containing 0.01% Tween20, the above-mentioned M2-purified sample was injected manually. While the absorbance at 280 nm was monitored, the elution peak at a retention volume of about 200 mL was collected in 2.5 mL each into 5-mL tubes. The collected fractions were combined, passed through a 0.22 µm filter (MILLIPORE, c/n: SLGV033RS), and then stored at 4°C.

After the activity of each lot of the purified diabody was examined, they were combined and concentrated to approximately 1 mg/mL using Centriprep YM-10 (amicon, c/n: 4304), passed through a 0.22 µm filter (MILLIPORE, c/n: SLGV033RS), and then stored.

### 7-3. Purification by chromatography (2)

From the culture supernatant obtained above (7-1), the C3B3 diabody was purified in three steps: ion exchange chromatography, hydroxyapatite chromatography, and gel filtration chromatography.

After a three-fold dilution of the culture supernatant with ultrapure water, the pH was adjusted to 8.0 using 1 M Tris. This was then subjected to a Q Sepharose Fast Flow column (GE Healthcare) equilibrated with 20 mM Tri-HCl (pH8.0) containing 0.02% Tween20, and the column was washed with the same buffer. Polypeptides adsorbed onto the column were then eluted using the same buffer with a linear concentration gradient of NaCl from 0 M to 0.5 M. The obtained fractions were analyzed by SDS-PAGE, and all fractions containing the C3B3 minibodies (C3B3 multimers and C3B3 diabody) were collected.

The C3B3 fraction obtained in the first step was added to a hydroxyapatite column (BIO-RAD, type I, 20 µm) equilibrated with 10 mM phosphate buffer (pH7.0) containing 0.02% Tween20, and after the column was washed with the same buffer, the concentration of phosphate buffer was increased linearly up to 250 mM for eluting the polypeptides adsorbed onto the column. The eluted peaks were analyzed by SDS-PAGE and gel filtration using Superdex 200 PC 3.2/30 column (GE Healthcare). Only the peak that shows the molecular weight of the desired C3B3 diabody was collected.

The C3B3 diabody peak fraction obtained in the second step was concentrated using amicon ultra 10 kDa cut (MILLIPORE), equilibrated in PBS(-) containing 0.01% Tween20, and then added to a HiLoad 26/60 Superdex 200 pg column (GE Healthcare). The obtained fractions were analyzed by SDS-PAGE, and the main peak containing the desired C3B3 diabody was determined to be the purified fraction.

When the purified C3B3 diabody was subjected to analytical gel filtration using Superdex 200 PC 3.2/30 column, it gave a single peak and the apparent molecular weight was approximately 52 kDa.

SDS-PAGE analysis of the C3B3 diabody showed that under both reducing and non-reducing conditions, a single band was observed at the position of the molecular weight of a monomer (approximately 27 kDa). Therefore, this showed that the C3B3 diabody is a dimer in which two molecules of single chain Fv are linked noncovalently.

### [Example 8] Evaluation of the efficacy of C3B3 diabody

### 8-1. Suppressive effects of the C3B3 diabody on in vitro cell growth

To analyze antitumor effects of the C3B3 diabody in detail, growth suppressive effects of the diabody on various human hematopoietic tumor cell lines were examined as follows.

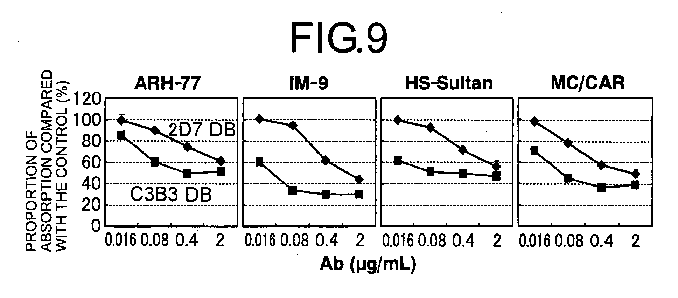

The cells used were human EBV-transformed B cell lines ARH-77, IM-9, and MC/CAR; and human Burkitt's lymphoma cell line, HS-Sultan. RPMI1640 medium containing 10% FCS was used to culture ARH-77, IM-9, and HS-Sultan. Iscove's modified Dulbecco's medium containing 20% FCS was used to culture MC/CAR. Cells were plated onto 96-well plates at a concentration of 3 x 10³ cells/well for ARH-77 and IM-9, 5 x 10³ cells/well for MC/CAR, and 1 x 10⁴ cells/well for HS-Sultan, and the cells were cultured in the presence of the C3B3 diabody or 2D7 diabody in a 5% CO₂ incubator at 37°C for three days. After WST-8 (Cat. No. 07553-15, Nakalai Tesque) was added to each well, culturing was continued for another four hours, and absorption at 450 nm (reference wavelength of 655 nm) was then measured using a microplate reader. The absorbance in wells with no antibody addition was defined as 100% and the absorbance in wells with no cell addition was defined as 0% to measure cell growth. Examination was carried out in triplicate and the mean and standard deviation was calculated (Fig. 9).

In all the cell lines used for the experiment, both the C3B3 diabody and 2D7 diabody demonstrated concentration-dependent cell growth suppression. However, in comparison, the C3B3 diabody showed growth-suppressing effects that surpass the maximum activity of 2D7 diabody with a lower concentration.

### 8-2. In vivo anti-tumor effects of the C3B3 diabody

### 8-2-1. Human IgG assay of mouse serum

The quantity of human IgG contained in mouse serum was determined by ELISA as described below. 100 µL of goat anti-human IgG (BIOSOURCE) diluted to 1 µg/mL with 0.1 mol/L bicarbonate buffer (pH9.6) was placed into a 96-well plate (Nunc). This was incubated at 4°C overnight, and the antibody was immobilized. After blocking, 100 µL of mouse serum diluted in a stepwise manner or 100 µL of human IgG (Cappel) as the standard sample was added. This was incubated at room temperature for two hours. After washing, 100 µL of a 5000-fold diluted alkaline phosphatase-labeled anti-human IgG antibody (BIOSOURCE) was added, and this was incubated at room temperature for two hours. After washing, substrate solution was added and incubated, and then absorbance was measured at 405 nm using MICROPLATE READER (BIO-RAD).

### 8-2-2. Anti-tumor effects of the C3B3 diabody on human EBV-transformed B cell (IM-9)-transplanted mice

### 8-2-2-1. Production of IM-9 transplanted mice

IM-9-transplanted mice were produced as follows. IM-9 cells subcultured *in vitro* in RPMI1640 medium (SIGMA-ALDRICH) containing 10% FCS (Hyclone) were adjusted to 5 x 10⁶ cells/mL in the above-mentioned medium. Scid mice (6-week-old female, Japan Clea) pretreated the previous day with intraperitoneal administration of 100 µL of anti-asialo-GM1 (Wako Pure Chemical Indurstries) was subjected to injection of 200 µL of the above-mentioned IM-9 cell preparation solution through the tail vein.

### 8-2-2-2. Antibody administration

For twice a day on the first, second, and third days after IM-9 transplantation for a total of six administrations, the antibody (2D7 diabody or C3B3 diabody) was administered to the above-mentioned IM-9-transplanted mice through the tail vein at 10 mg/kg. For the control group, PBS containing Tween20 was administered through the tail vein at 10 mL/kg.

### 8-2-2-3. Evaluation of the C3B3 diabody anti-tumor effects on IM-9-transplanted mice

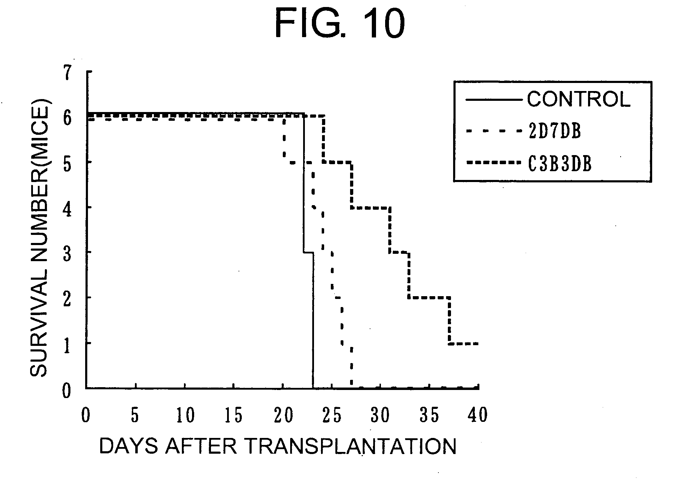

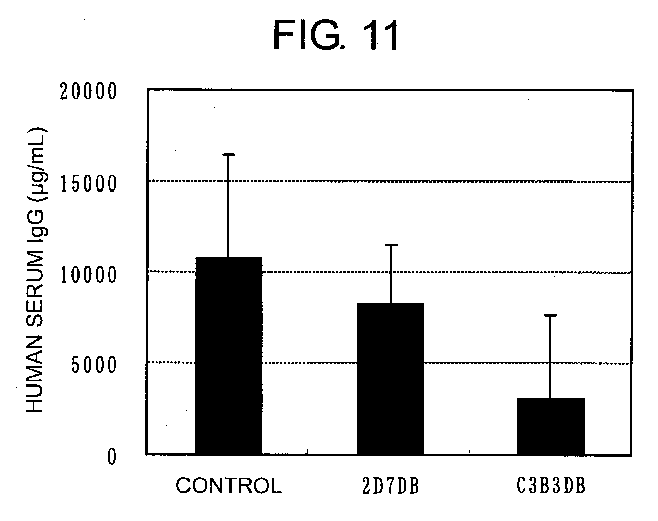

Anti-tumor effects of the C3B3 diabody were evaluated using the survival time of the mice and the amount of human IgG in the serum. As shown in Fig. 10, the survival time of C3B3 diabody-administered mice was clearly extended compared to the control group mice. The survival time was extended even when compared with the 2D7 diabody-administered mice. Furthermore, on the 14th day after IM-9 transplantation, sera were collected from the mice, and measurements were made by ELISA as described above in 8-2-1 (Fig. 11). As a result, an obvious reduction in the serum human IgG level was observed in the C3B3 diabody-administered mice when compared with the control group mice. The serum human IgG level showed a decreasing tendency in the C3B3 diabody-administered mice even in comparison with the 2D7 diabody-administered mice. Therefore, this showed that the C3B3 diabody has a stronger antitumor effect on human EBV-transformed B cell-transplanted mice than the 2D7 diabody.

### [Example 9] Examination of the cell death-inducing action of the C3B3 diabody on human PBMC

The cell death-inducing effect of the C3B3 diabody and 2D7 diabody on human peripheral blood mononuclear cells (PBMCs) was examined. PBMCs were isolated from the peripheral blood of a healthy adult volunteer by specific gravity centrifugation. The PBMCs were plated on to a 96-well plate at 5 x 10⁴ cells/well (in the case of concanavalin A stimulation) or at 1.5 x 10⁵ cells/well (in the case of SAC stimulation). Concanavalin A (hereinafter referred to as ConA, Wako Pure Chemical Industries) was added at a final concentration of 10 µg/mL, and SAC (Pansorbin Cells, Carbiochem) was added at a final concentration of 0.01%. Furthermore, the C3B3 diabody or 2D7 diabody was added at a final concentration of 10 µg/mL. Cells were cultured in a 5% CO₂ incubator at 37°C for three days. On the third day of culturing, 10 µL of Cell Counting Kit-8 (Dojindo) was added to each well, and after 7 hours of reaction in a 5% CO₂ incubator at 37°C, absorbance at 450 nm (reference wavelength of 630 nm) was measured using a MICROPLATE READER (BIO-RAD).

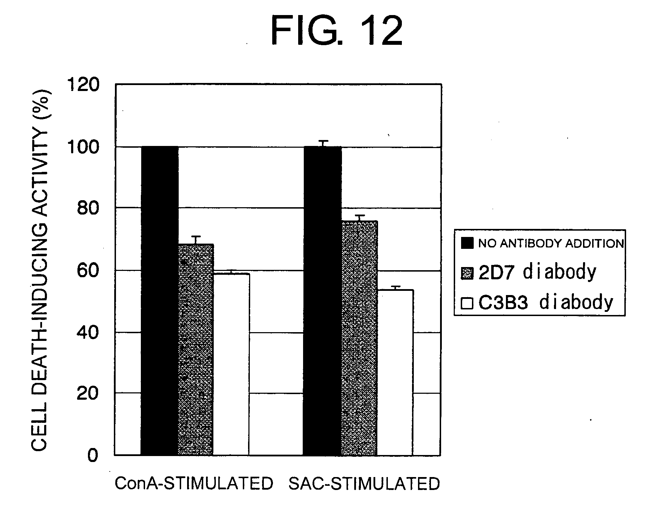

As shown in Fig. 12, the results showed that the C3B3 diabody shows stronger cell death-inducing activity than the 2D7 diabody when stimulated with ConA as well as when stimulated with SAC.

### [Example 10] In vitro cell-growth suppressive effects of the C3B3 diabody

The C3B3 diabody's growth suppressive effects on human T-cell tumor cells were examined as follows.

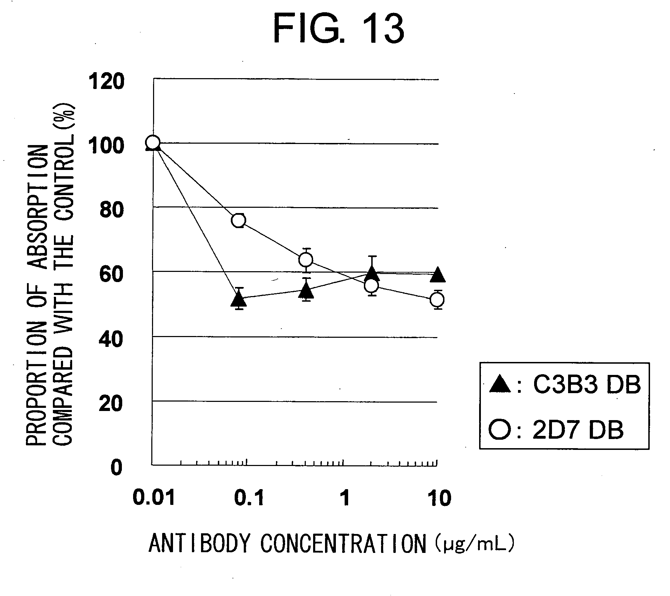

Cells of Jurkat (E6-1) strain (purchased from ATCC) were used. RPMI1640medium containing 10% FCS was used for culturing the Jurkat (E6-1) cells. Jurkat cells were plated on to a 96 well plate at 2×10⁴ cells/well and cultured in a 5%CO₂ incubator at 37°C for 3 days under the presence of the C3B3 diabody or 2D7 diabody. Next, Cell Counting Kit-8 (Code. No. CK04, Dojindo Laboratories, Japan) was added to each well. After incubating for two hours, absorbance at 450nm (reference wave length of 630nm) was measured using a microplater reader. To measure cell growth, the absorbance in wells with no antibody addition was defined as 100% and absorbance in wells with no cell addition was defined as 0%. Examination was carried out in triplicates and the mean and standard error (SE) was calculated (Fig. 13). Both the C3B3 diabody and 2D7 diabody demonstrated concentration-dependent cell growth suppression in Jurkat cells. However, in comparison, the C3B3 diabody showed a higher growth-suppressing effect than that of 2D7 diabody even at a lower concentration.

Previous studies have shown that HLA antibodies show effects on lymphocytes in general (WO2004/033499 and WO2005/100560), and full-length antibodies and low-molecular-weight antibodies newly discovered in the present invention according to the above-mentioned results are expected to generally show effects on lymphocytes.

### Industrial Applicability

The present invention provides novel anti-HLA-A antibody and C3B3 antibody which have cell death-inducing activity when cross-linked with an anti-mouse IgG antibody. Low -molecular-weight antibodies of the C3B3 antibody (diabodies) showed strong cell death-inducing activity without the addition of an anti-mouse IgG antibody, and their activity greatly exceeded the activity of conventional low-molecular-weight antibodies in an *in vitro* tumor cell assay system. Furthermore, the low-molecular-weight antibodies also showed higher anti-tumor effects than conventional low-molecular-weight antibodies in *in vivo* tumor-transplanted model mice. More specifically, low-molecular-weight C3B3 antibodies are superior to conventional low-molecular-weight antibodies in that they show high cytocidal activity against hematopoietic tumor cells, and at the same time, they show cell death-inducing activity at lower concentration. Therefore, the low-molecular-weight antibodies can be expected to exert superior drug efficacy than conventional low-molecular-weight antibodies as therapeutic agents against hematopoietic tumors, myeloid immunological disorders, autoimmune diseases, and the like.

## Claims

1. An antibody comprising heavy chain variable regions that comprise CDR1, 2, and 3 consisting of the amino acid sequences of SEQ ID NOs 7, 8, and 9.

2. An antibody comprising light chain variable regions that comprise CDR1, 2, and 3 consisting of the amino acid sequences of SEQ ID NOs 10, 11, and 12.

3. An antibody comprising heavy chain variable regions that comprise CDR1, 2, and 3 consisting of the amino acid sequences of SEQ ID NOs 7, 8, and 9, and light chain variable regions that comprise CDR1, 2, and 3 consisting of the amino acid sequences of SEQ ID NOs 10, 11, and 12.

4. An antibody comprising the heavy chain variable region of any one of:
(a) a heavy chain variable region comprising the amino acid sequence of SEQ ID NO: 2;
(b) a heavy chain variable region that comprises an amino acid sequence with one or more amino acid substitutions, deletions, insertions, and/or additions in the amino acid sequence of SEQ ID NO: 2, and which is functionally equivalent to the heavy chain variable region of (a);
(c) a heavy chain variable region comprising an amino acid sequence encoded by a DNA comprising the nucleotide sequence of SEQ ID NO: 1; and
(d) a heavy chain variable region comprising an amino acid sequence encoded by a DNA that hybridizes under stringent conditions with a DNA comprising the nucleotide sequence of SEQ ID NO: 1.

5. An antibody comprising the light chain variable region of any one of:
(e) a light chain variable region comprising the amino acid sequence of SEQ ID NO: 4;
(f) a light chain variable region that comprises an amino acid sequence with one or more amino acid substitutions, deletions, insertions, and/or additions in the amino acid sequence of SEQ ID NO: 4, and which is functionally equivalent to the light chain variable region of (e);
(g) a light chain variable region comprising an amino acid sequence encoded by a DNA comprising the nucleotide sequence of SEQ ID NO: 3; and
(h) a light chain variable region comprising an amino acid sequence encoded by a DNA that hybridizes under stringent conditions with a DNA comprising the nucleotide sequence of SEQ ID NO: 3.

6. An antibody comprising a heavy chain variable region of any one of:
(a) a heavy chain variable region comprising the amino acid sequence of SEQ ID NO: 2;
(b) a heavy chain variable region that comprises an amino acid sequence with one or more amino acid substitutions, deletions, insertions, and/or additions in the amino acid sequence of SEQ ID NO: 2, and which is functionally equivalent to the heavy chain variable region of (a);
(c) a heavy chain variable region comprising an amino acid sequence encoded by a DNA comprising the nucleotide sequence of SEQ ID NO: 1; and
(d) a heavy chain variable region comprising an amino acid sequence encoded by a DNA that hybridizes under stringent conditions with a DNA comprising the nucleotide sequence of SEQ ID NO: 1;
and a light chain variable region of any one of:
(e) a light chain variable region comprising the amino acid sequence of SEQ ID NO: 4;
(f) a light chain variable region that comprises an amino acid sequence with one or more amino acid substitutions, deletions, insertions, and/or additions in the amino acid sequence of SEQ ID NO: 4, and which is functionally equivalent to the light chain variable region of (e);
(g) a light chain variable region comprising an amino acid sequence encoded by a DNA comprising the nucleotide sequence of SEQ ID NO: 3; and
(h) a light chain variable region comprising an amino acid sequence encoded by a DNA that hybridizes under stringent conditions with a DNA comprising the nucleotide sequence of SEQ ID NO: 3.

7. An antibody comprising the amino acid sequence of any one of:
(a) the amino acid sequence of SEQ ID NO: 6;
(b) an amino acid sequence with one or more amino acid substitutions, deletions, insertions, and/or additions in the amino acid sequence of SEQ ID NO: 6;
(c) an amino acid sequence encoded by a DNA comprising the nucleotide sequence of SEQ ID NO: 5; and
(d) an amino acid sequence encoded by a DNA that hybridizes under stringent conditions with a DNA comprising the nucleotide sequence of SEQ ID NO: 5.

8. An antibody that binds to a same epitope as the epitope of the human leukocyte antigen (HLA) protein to which the antibody of any one of claims 1 to 7 binds.

9. The antibody of any one of claims 1 to 8, which is a monoclonal antibody.

10. The antibody of any one of claims 1 to 9 that recognizes a human leukocyte antigen (HLA).

11. The antibody of claim 10, wherein the HLA is an HLA class I.

12. The antibody of claim 11, wherein the HLA class I is an HLA-A.

13. The antibody of any one of claims 1 to 12, which is a low-molecular weight antibody.

14. The antibody of claim 13, wherein the low-molecular-weight antibody is a diabody.

15. A polynucleotide of (a) or (b):
(a) a polynucleotide comprising the nucleotide sequence of SEQ ID NO: 1, 3, or 5; or
(b) a polynucleotide that hybridizes under stringent conditions with the polynucleotide of (a), and encodes an antibody having an activity equivalent to the antibody of any one of claims 1 to 14.

16. A vector comprising the polynucleotide of claim 15.

17. A host cell that comprises the polynucleotide of claim 15 or the vector of claim 16.

18. A method for producing the antibody of any one of claims 1 to 14, wherein the method comprises the steps of:
(a) producing the polynucleotide of claim 15;
(b) constructing a vector comprising the polynucleotide of (a);
(c) introducing the vector of (b) into host cells; and
(d) culturing the host cells of (c).

19. A cell death-inducing agent comprising the antibody of any one of claims 1 to 14 as an active ingredient.

20. The cell death-inducing agent of claim 19 which induces cell death of a B cell or T cell.

21. The cell death-inducing agent of claim 20, wherein the B cell or T cell is an activated B cell or activated T cell.

22. A cell growth-suppressing agent comprising the antibody of any one of claims 1 to 14 as an active ingredient.

23. An antitumor agent comprising the antibody of any one of claims 1 to 14 as an active ingredient.

24. The antitumor agent of claim 23, wherein the tumor is hematopoietic tumor.

25. A therapeutic agent for autoimmune diseases which comprises the antibody of any one of claims 1 to 14 as an active ingredient.
